# EUROPEAN PATENT SPECIFICATION

(11) **EP 2 831 593 B1**
(45) Date of publication and mention of the grant of the patent: **30.05.2018**
(21) Application number: 13770052.2
(22) Date of filing: 15.03.2013
(51) Int. Cl.: G01N 33/574, G01N 33/68, G01N 33/48

(54) **S100A8/A9 AS A DIAGNOSTIC MARKER AND A THERAPEUTIC TARGET**
S100A8/A9 ALS DIAGNOSTISCHER MARKER UND THERAPEUTISCHES TARGET
S100A8/A9 À TITRE DE MARQUEUR DIAGNOSTIQUE ET DE CIBLE THÉRAPEUTIQUE

(30) Priority: 30.03.2012 US 201261618357 P
(43) Date of publication of application: 04.02.2015
(73) Proprietor: Sloan Kettering Institute For Cancer Research, New York, NY 10065 (US)
(72) Inventor: MASSAGUE, Joan, New York New York 10065 (US); ACHARYYA, Swarnali, New York New York 10044 (US)
(74) Representative: Chapman IP
(86) International application number: PCT/US2013/032617
(87) International publication number: WO 2013/148370

(56) References cited:
- WO-A2-2008/127526
- WO-A2-2012/038068
- US-A1- 2008 268 435
- US-A1- 2008 268 435
- US-A1- 2011 027 275
- GEETHA SRIKRISHNA: "S100A8 and S100A9: New Insights into Their Roles in Malignancy", JOURNAL OF INNATE IMMUNITY, vol. 4, no. 1, 6 September 2011 (2011-09-06), pages 31-40, XP55222065, CH ISSN: 1662-811X, DOI: 10.1159/000330095
- BIECHE ET AL.: 'CXC CHEMOKINES LOCATED IN THE 4Q21 REGION ARE UP-REGULATED IN breast cancer' ENDOCRINE-RELATED CANCER vol. 14, no. 4, 2007, pages 1039 - 1052, XP055165948
- MOON ET AL.: 'Global gene expression profiling unveils S100A8/A9 as candidate markers in H-Ras-mediated human breast epithelial cell invasion' MOLECULAR CANCER RESEARCH vol. 6, no. 10, October 2008, pages 1544 - 1553, XP002602436
- ACHARYYA ET AL.: 'A CXCL1 paracrine network links cancer chemoresistance and metastasis' CELL vol. 150, no. 1, 06 July 2012, pages 168 - 178, XP028400992
- Won Suk Yang ET AL: "Proteomic Approach Reveals FKBP4 and S100A9 as Potential Prediction Markers of Therapeutic Response to Neoadjuvant Chemotherapy in Patients with Breast Cancer", JOURNAL OF PROTEOME RESEARCH., vol. 11, no. 2, 3 February 2012 (2012-02-03), pages 1078-1088, XP055252574, US ISSN: 1535-3893, DOI: 10.1021/pr2008187

## Description

### Field of the Invention

This application relates to methods and kits as defined in the appended claims.

### Background of the Invention

Breast cancer remains the most common malignant disease in women with one million new cases being diagnosed annually worldwide, causing 400,000 deaths per year (Gonzalez-Angulo et al., 2007). The vast majority of these deaths are due to metastatic disease. Indeed, although the five-year disease free survival rate is 89% in well-treated localized breast cancer patients, the appearance of metastatic disease is almost always a harbinger of eventual cancer mortality. The median survival of patients with stage IV breast cancer is between one and two years, and only a quarter of such patients survive five or more years from diagnosis of distant metastases. (Jones, 2008). Hence, efforts to better understand and control breast cancer metastases are imperative.

The two established forms of systemic therapy for metastatic disease are hormonal treatments for hormone-dependent (estrogen and/or progesterone receptor positive) cases and cytotoxic chemotherapy for cases without hormone receptors. In addition, hormone-dependent breast cancers almost always become refractory to initially effective hormonal treatments, thus eventually requiring chemotherapy as well. Trastuzumab, an antibody to the extracellular domain of the c-erbB2/HER2 receptor tyrosine kinase, often augments the chemotherapy effect in cases over-expressing this gene (Hudis, 2007). The role of antiangiogenic therapy as a supplement to chemotherapy is under active evaluation (Bergers and Hanahan, 2008; Ebos and Kerbel, 2011). While tumor shrinkage is commonly accomplished on initial use of chemotherapy, the eventual emergence of drug resistance and resulting tumor re-growth in the original sites of involvement as well as new sites is the rule (Jones, 2008). Estrogen receptor negative breast cancers in particular have a predilection to grow rapidly and to involve visceral organs such as the lung (Hess et al., 2003). On progressive disease from initial chemotherapy, different chemotherapy drugs are then usually offered, but the odds of response to subsequent administrations of chemotherapy declines with each episode of response and progression. Ultimately, pan-resistance occurs, which in association with the progression of metastatic spread, an almost universally linked process, is the cause of death (Gonzalez-Angulo et al., 2007).

Research directed to the treatment of cancer, including breast cancer, is continuing to produce a variety of new therapeutic targets and approaches. The potential for pan-resistance following treatment with various drugs as well as the availability of numerous alternatives makes it desirable to be able to perform tests prior to therapy to select the treatment that would be most likely to be effective for a specific patient's cancer. In addition, it would be desirable to be able to monitor the progress of therapeutic treatment so that a change to a different treatment could be considered if the cancer developed resistance to a treatment modality selected. The design of meaningful tests of this type, however, requires a sound understanding of the basis for the activity of the chemotherapy agent, as well as the manner in which resistance may arise.

Drug resistance in cancer can be based on cancer cell-autonomous functions. For example, secondary mutations or compensatory activation of feedback and bypass pathways are responsible for resistance to various drugs that target driver oncogenes (Bean et al., 2008; Johannessen et al., 2010; Nazarian et al., 2010; Poulikakos et al., 2010; Shah et al., 2002; Villanueva et al., 2010). A combination of host and tumor mediated pathways can lead to resistance to anti-angiogenic drugs (Ebos et al., 2009; Paez-Ribes et al., 2009; Shojaei et al., 2007). In the case of chemotherapeutic agents, resistance develops due to both intrinsic mechanisms as well as those acquired *de-novo* during the course of the treatment (Gonzalez-Angulo et al., 2007). Recent evidence points at tumor microenvironment components such as macrophages and endothelial cells as potential participants in the generation of chemoresistance (DeNardo, 2011; Gilbert and Hemann, 2010; Joyce and Pollard, 2009; Shaked et al., 2008). However, an integrated understanding of acquired drug resistance in the context of inputs from tumor and its microenvironment is lacking. Such insights could be critical for designing more effective therapies that can overcome resistance and improve outcome from a palliative to curative clinical response in cancer.

CXCL1/2 expression has been associated with breast cancer but the exact nature of this significance is not clear. *CXCL1 is* among a set of 18 genes that can predict whether a primary tumor will relapse to lungs, and *CXCL1 is* the only inflammatory chemokine gene in this set (Minn et al., 2005). Furthermore, aggressive tumor cells that have colonized distant organs and have the potential of re-infiltrating primary tumors, so-called "self-seeders" also significantly upregulate *CXCL1* (Kim et al., 2009). Additionally, breast cancer patients resistant to chemotherapeutic drugs showed a gain of 4q21, a region that harbors *CXCL1* and other closely related chemokines of the CXC-family such as *CXCL2* (Fazeny-Dorner et al., 2003).

Drugs that target the receptor associated with CXCL1/2 (CXCR) have been developed and are currently undergoing evaluation. These include small molecule inhibitors such as reperixin (formerly repartaxin) ((*R*(-)-2-(4-isobutylphenyl)propionyl methansulphonamide) with a pharmaceutically acceptable counterion); and *N*-(2-hydroxy-4-nitrophenyl)-*N'*-phenylurea and *N*-(2-hydroxy-4-nitrophenyl)-*N'*-(2-bromophenyl)urea (White et al., 1998, The Journal of Biological Chemistry, 273, 10095-10098.). See also WO2005/113534 and WO2005/103702.

### Summary of the Invention

A method of selecting patients having lung or breast cancer that are suitable for a defined treatment, and a kit consisting of reagents for assessing responsiveness to chemotherapy treatments are provided substantially as described herein and according to the appended claims.

The present inventors have determined that cytotoxic chemotherapy with agents commonly employed in both the adjuvant setting and advanced-disease can paradoxically trigger pro-survival cascades through tumor-stroma interactions, thereby leading to drug resistance. Through mechanistic and clinical evidence, the inventors have identified TNFα-CXCL1/2-S100A8/A9 as a new paracrine survival axis that is activated upon chemotherapy treatment. S100A8/A9 proteins provide a valuable diagnostic marker of the activation of this survival axis, which can be used to guide the selection of standard of care therapeutics, or therapeutics that target this survival axis, including in particular therapeutics that target the chemokines CXCL1/2/3/8 or their receptors CXCR1/2, the cytokine TNF-alpha or its receptor TNFR, or the factors S100A8/A9 or their receptors RAGE and TLR4, for use in treating the patient. These therapeutics can be used in combination with other agents, since it is shown that pharmacological targeting of CXCL1/2 paracrine interactions significantly improves chemotherapy response and reduces metastasis.

In addition, S100A8/A9 proteins can be used as a prognostic marker for response to standard of care chemotherapy and for potential response to treatments with therapeutics that target and inhibit S1 00A8/A9 or their receptors, CXCL1/2/3/8 or their receptors, or TNF-alpha or its receptors.

Thus, the invention also provides a method for treatment using an appropriate chemotherapeutic agent for a given patient. The purpose of administering this therapy would be to decrease the ability of the cancer cells to use S100A8/A9 as a defense against chemotherapy. As a result, chemotherapy would be more effective at reducing the tumor. Therefore, the therapy claimed here would make chemotherapy more effective, in achieving the eradication of a tumor. Furthermore, the therapy proposed here can allow a decrease in the dose of chemotherapy and still achieve the same beneficial effect with less toxicity. The invention further provides a kit for providing a prognostic evaluation through the evaluation of S100A8/A9 proteins in a patient.

### Brief Description of the Drawings

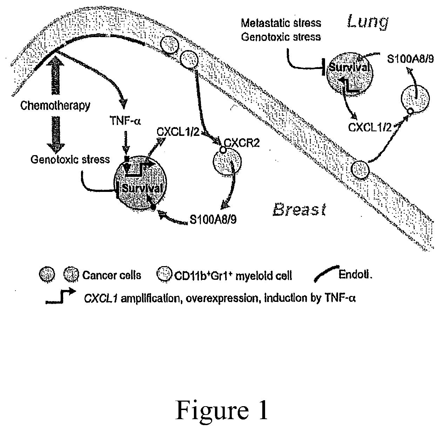

Figure 1 depicts a model showing how CXCL1 paracrine interactions promote resistance to chemotherapy and metastasis in breast tumors and lung microenvironment. Genotoxic agents such as doxorubicin, cyclophosphamide and paclitaxel limit the survival of cancer cells but also increase TNF-α production from endothelial cells. TNF-α enhances CXCL1/2 expression in cancer cells. Other modes of CXCL1/2 upregulation in cancer cells include 4q21 amplification and overexpression. CXCL1/2 from cancer cells recruit CD11b+Gr1+ myeloid cells that express CXCR2 (receptors for CXCL1/2). Myeloid cells recruited by CXCL1/2 thereby enhance viability of cancer cells through S100A8/A9 factors.

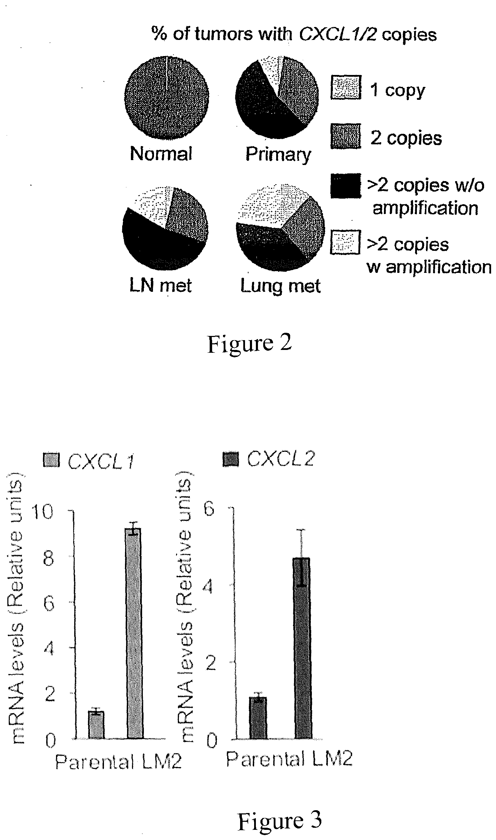

Figure 2 (formerly S1A) shows quantification of *CXCL1*/*2* copies determined by FISH analysis in TMA from breast cancer patients. n=11 (Normal), n=40 (Primary tumors), n=30 (Lymph node metastases, LN met), n=26 (Lung metastases, Lung met).
Figure 3 shows expression of *CXCL1* and *CXCL2* in MDA-MB-231 breast cancer cells (parental) and lung metastatic derivative MDA231-LM2 (LM2) cells determined by qRT-PCR. Data are averages ± SEM from two independent experiments.

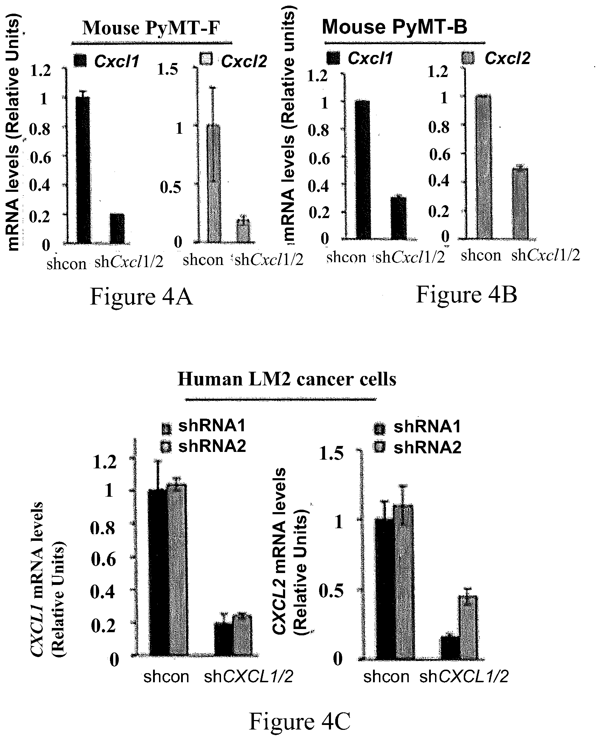

*Figures 4A-4C* *show CXCL1* and *CXCL2* expression in control (sh-con) and *CXCL1*/*2* knockdown cells in mouse PyMT cells (4A and 4B) and human LM2 cells (4C). Two independent sublines of PyMT tumor cells derived from MMTV-PyMT transgenic mice in FVB/N (PyMT-F for short) and C57/BL6 backgrounds (PyMT-B for short) are shown. Two independent short hairpin RNAs (shRNA1 and shRNA2) targeting *CXCL1*/*2* tested in LM2 cells are shown in Figure 4C. Expression was determined by qRT-PCR in two independent experiments. Data are average ± SEM.

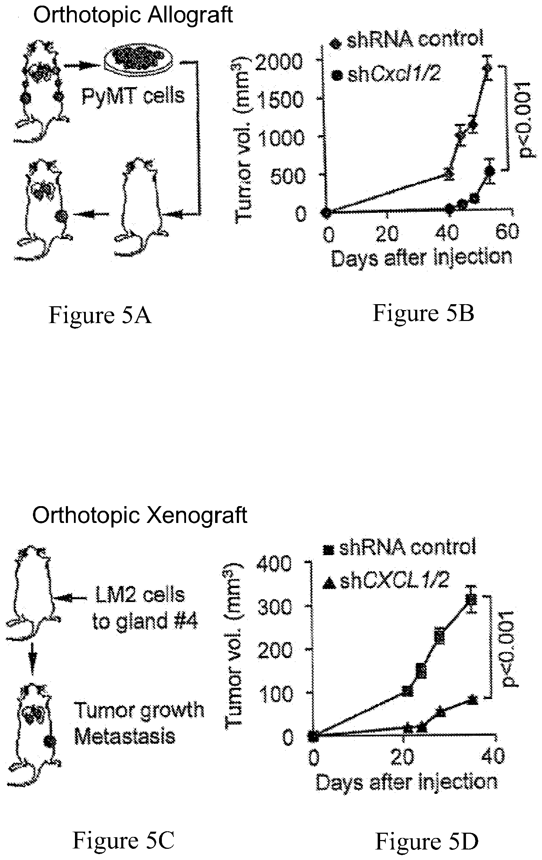

Figures 5A and B relate to breast cancer progression in orthotopic PyMT isograft mouse models. Figure 5A shows a schematic representation of syngeneic metastasis model. PyMT mammary cancer cells were isolated from MMTV-PyMT mammary tumors, transduced with shRNA control or sh*Cxcl1*/*2* and transplanted into syngeneic mice. Fig. 5B shows growth curves of tumors from control and sh*Cxcl1*/*2* PyMT-F cells. Tumor size was measured at the indicated times using a digital caliper. Data are averages ± SEM. n=6 mice per group. P values determined by Student's t-test.
Figures 5C and D relate to breast cancer progression in orthotopic LM2 xenograft model. Fig. 5C shows a schematic representation outlining the xenograft metastasis model. LM2 metastatic breast cancer cells were implanted into immunodeficient NOD-SCID mice. Mammary tumor growth and lung metastasis were determined. Fig. 5D shows growth curves of tumors from LM2 cells transduced with control or *CXCL1*/*2* shRNA. Tumor size was measured at the indicated times using a digital caliper. Data are averages ± SEM. Control n=13, sh*CXCL1*/*2* n=7. P values were determined by Student's t-test.

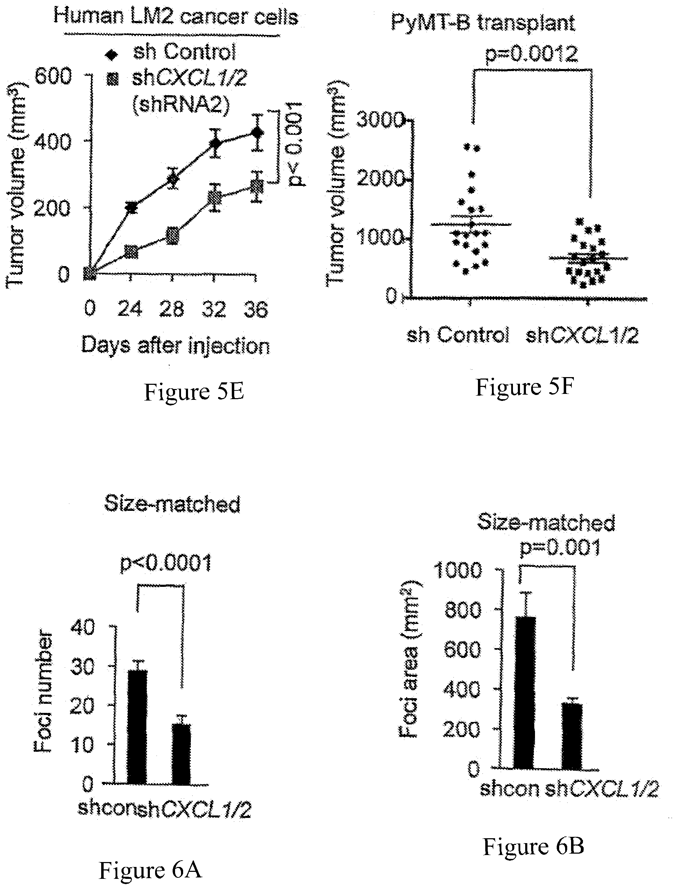

Figure 5E shows growth of mammary tumors derived from LM2 cells expressing either control or sh*CXCL1*/*2* in orthotopic metastasis assay (second set of hairpin). Data are shown as averages ± SEM. n=10 per group. P values determined by Student's t-test.
Figure 5F shows distribution of mammary tumor volume (mm3) from tumor cells derived from PyMT-B mice expressing either control or sh*CXCL1*/*2* in orthotopic metastasis assay on day 19 post tumor inoculation. Data are shown as averages ± SEM. n=20 tumors per group. P values determined by Student's t-test.
Figures 6A and B relates to lung metastasis in a xenograft mouse model determined by automated counting of metastatic foci area or foci number. Metastasis was determined in mice where control and sh*CXCL1*/*2* LM2 tumors were size matched. Data are averages ± SEM. n=5 mice per group. P values determined by Student's t-test.

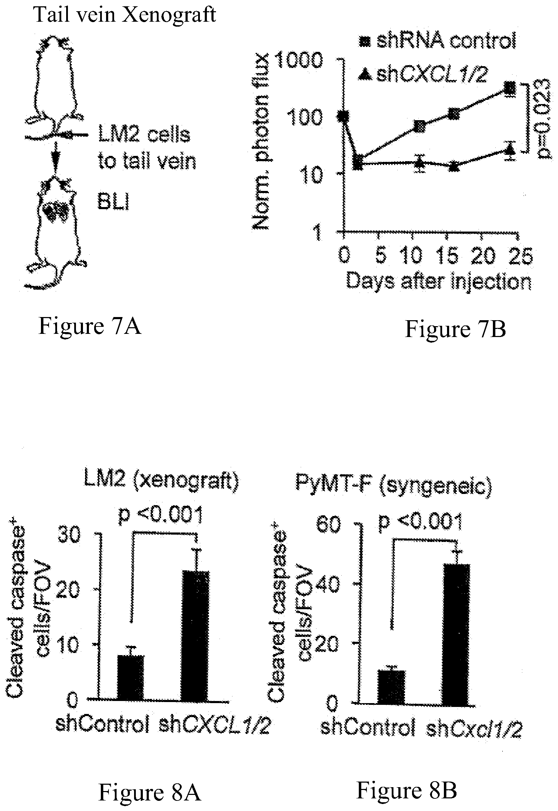

Figures 7A and B relate to lung colonization of MDA231-LM2 cells transduced with control and sh*CXCL1*/*2.* Figure 7A shows a schematic representation of lung colonization assay. Luciferase labeled LM2 cancer cells were injected intravenously and monitored over time by non-invasive bioluminescence imaging (BLI). Figure 7B shows BLI quantification of lung colonization ability of control and sh*CXCL1*/*2* LM2 cells. Data are averages ± SEM. n=7 per group. P values determined by Student's t-test.
Figures 8A and B relates to quantification of apoptosis in mammary tumors analyzed by Cleaved caspase-3 staining. Mouse mammary glands were injected with LM2 cells (Figure 8A) or PyMT-F cells (Figure 8B) expressing shRNA control or sh*CXCL1*/*2* and analyzed at endpoint (LM2, 6 weeks; PyMT-F, 9 weeks after tumor implantation). Scale bar equals 30µm. Data are averages ± SEM. n=4 mice per group. P values were calculated by Student's t-test.

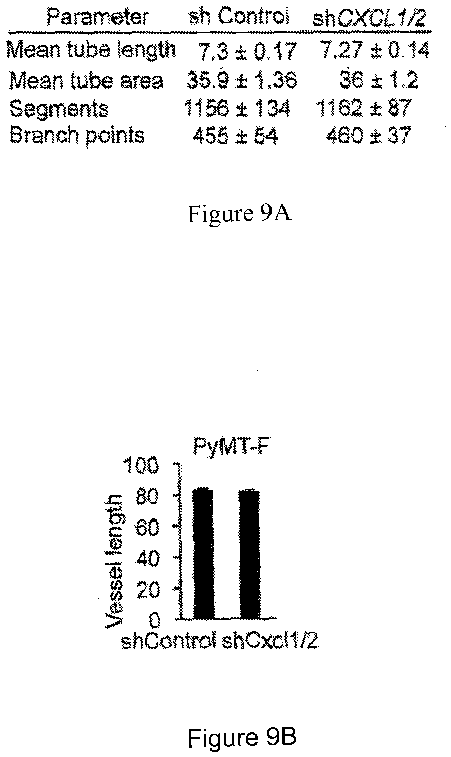

Figures 9A and B relate to automated morphometric analysis of tumor vessels detected by immunostaining of endothelial marker CD34. Control and sh*CXCL1*/*2* mammary tumors (LM2, A or PyMT-F, B) were analyzed at endpoint (6 and 9 wks post tumor inoculation, respectively). Data are shown as averages ± SEM. n= 4-6 mice per group. P values determined by Student's t-test.

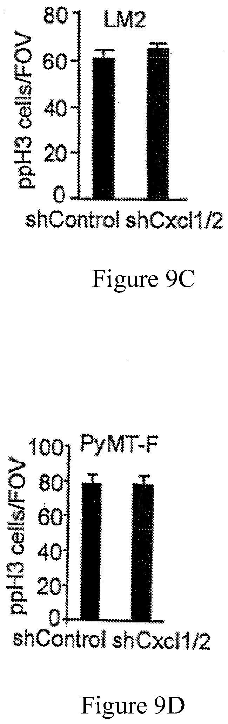

Figures 9C and D relate to proliferation in control and sh*CXCL1*/*2-* LM2 or PyMT-F mammary tumors, respectively, determined by phosphohistone H3 (ppH3) immunostaining at endpoint (6 and 9 wks post tumor inoculation, respectively). Data are shown as average ± SEM; n=5 mice per group. P values determined by Student's t-test.

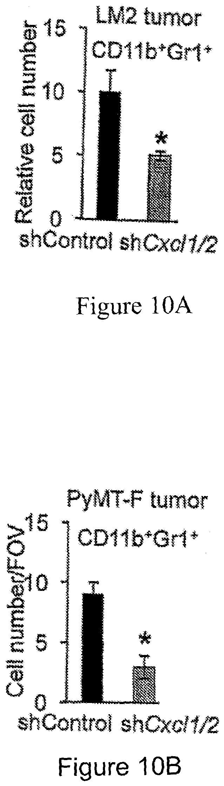

Figures 10A and B show the relative numbers of CD11b+Gr1 + cells in tumors from LM2 or PyMT-F tumor cells expressing either shRNA control or sh*CXCL1*/*2* quantitated by a combination of magnetic and flow sorting in 10A and immunostaining analysis in 10B.

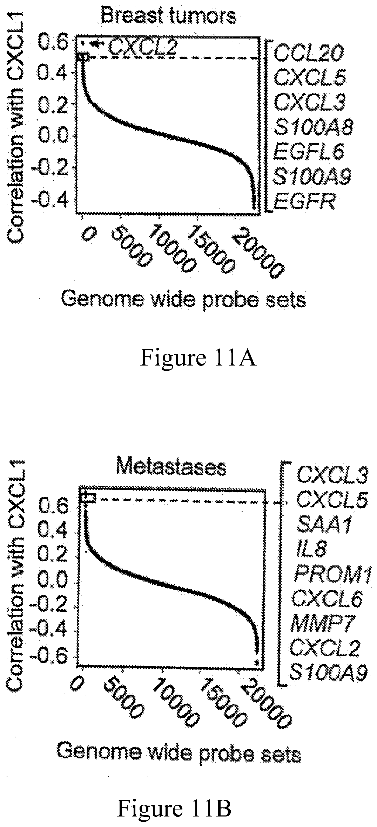

Figures 11A and 11B show gene ranking according to correlation with *CXCL1* expression. Expression data from three independent primary breast cancer microarray datasets and from breast cancer metastases datasets were used. Genes were filtered based on extracellular localization to identify paracrine mediators. Gene list on the right shows genes that correlate highest with *CXCL1.* Complete lists of genes that correlate with CXCL1 with a correlation coefficient >0.3 in the primary breast cancer and metastases datasets are included in the Tables.

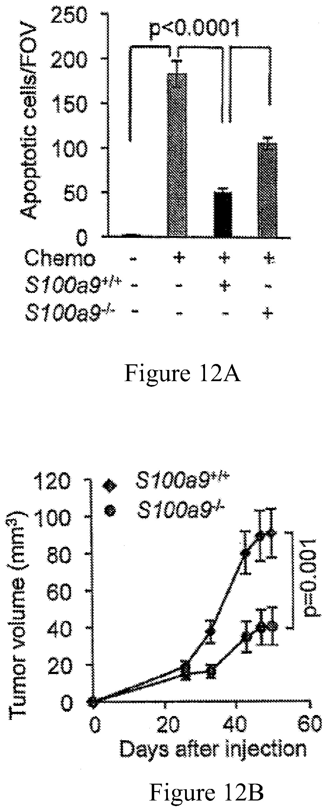

Figure 12A shows the results of TUNEL analysis detecting apoptotic cancer cells in co-culture assay. LM2 cancer cells were cultured alone or overnight in the presence of *S100a9*+/+ or *S100a9*-/- bone marrow cells and subsequently treated with chemotherapeutic drug (Chemo), doxorubicin (0.8µM). Data are average ± SEM of triplicates. P values determined by Student's t-test.
Figure 12B shows LM2 tumor growth curves in mice transplanted with either *S100a9+*/*+* or *S100a9-*/*-* bone marrow. Data points show averages ± SEM. n=19 tumors per group. P value was determined by Student's t-test

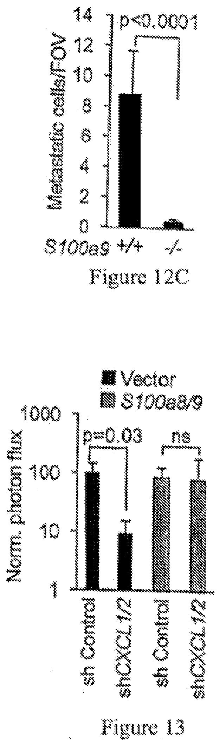

Figure 12C shows quantitation of lung metastasis at 60 days after inoculation of LM2 tumors, in mice that were transplanted with *S100a9*+/+or *S100a9-*/*-* bone marrow. Scale bar equals 60µm. Data points show averages ± SEM. n=4-6 mice per group. P value was determined by Student's t-test.
Figure 13 relates to lung colonization by LM2 cells transduced with control shRNA or sh*CXCL1*/*2,* with or without ectopic expression of *S100A8*/*A9.* Lung colonization was assessed by non-invasive bioluminescence imaging (BLI) at 4 weeks after tail vein injection of the cells. Figure 13 shows normalized BLI quantification represented by photon flux of lung colonization ability.

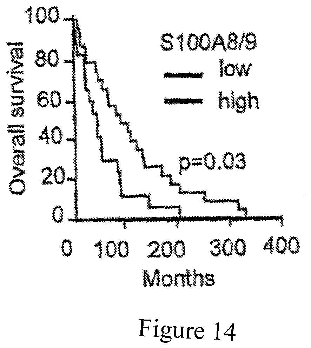

Figure 14 is a Kaplan-Meier plot reflecting overall survival analysis on breast cancer patients classified according to total S100A8/A9 expression in lung metastasis. High S100A8/A9 levels correlate with poor overall survival in breast cancer patients with lung metastases as determined by Kaplan-Meier analysis. n=23 for S100A8/A9 low group, n=17 for S100A8/A9 high group. P-values were calculated by log-rank test.

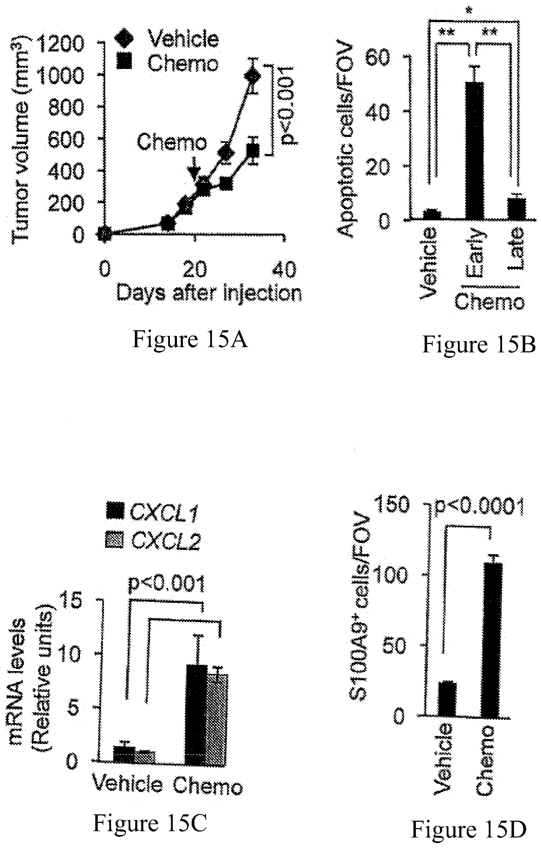

Figure 15A shows tumor growth in mice treated with saline vehicle or a combination of doxorubicin and cyclophosphamide chemotherapy (AC chemo). The treatment was initiated once LM2 tumors reached 300 mm3 and was repeated once weekly. Data represent averages ± SEM. n=6-8 mice per group. P values were determined by Student's t-test.
Figure 15B shows apoptosis determined by TUNEL staining in tumors treated with vehicle or AC chemotherapy for 3 days (*early*) or 8 days (*late*) both using the same treatment regimen. Data represent averages ± SEM. n=3-5 mice per group. P values were calculated by Student's t-test. *p=0.02, **p<0.0001.
Figure 15C shows *CXCL1*/*2* expression in whole tumors harvested from mice treated with saline vehicle or AC chemotherapy for 8 days. Data represent averages±SEM. n=6-8 mice per group. P values were determined by Student's t-test.
Figure 15D shows quantitation of S100A9 positive cells in tumors from control and AC chemotherapy treated mice (prolonged treatment). Data presented are average numbers of S100A9 positive cells per field of view (FOV) ± SEM. n=4-5 mice/group. Data representative of three independent experiments.

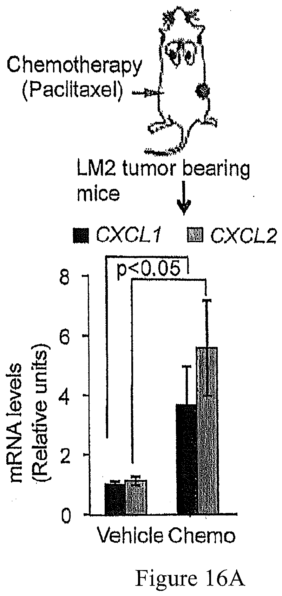

Figure 16A shows a shematic diagram of chemotherapy treatment and *CXCL1*/*2* expression in mammary LM2 tumors from mice treated with paclitaxel chemotherapy weekly. Data represent average expression ± SEM. n=5 mice per group. P value was determined by Student's t-test.

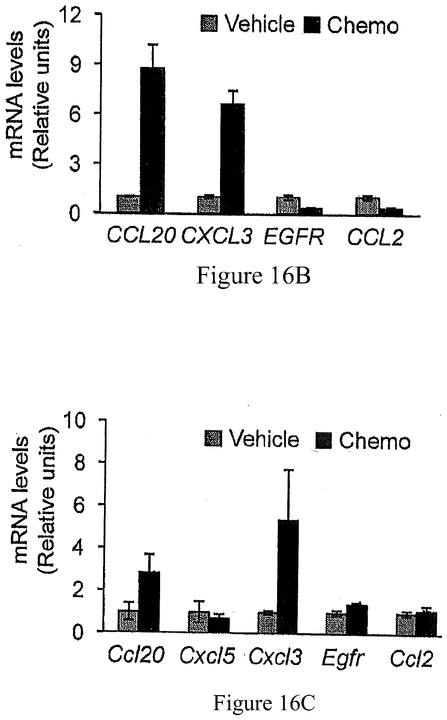

Figures 16B and C show gene expression analysis of *CXCL1* associated genes shown in Table 5. Human specific primers (16B) or mouse specific primers (16C) used in qRT-PCR comparing control (vehicle) and AC chemotherapy treated (chemo) tumors. There was no detectable expression of *CXCL5, EGFL6, CCL18* using human primers and *Egfl6* for mouse primers. Data represent average expression ± SEM. n=5-11 mice per group.

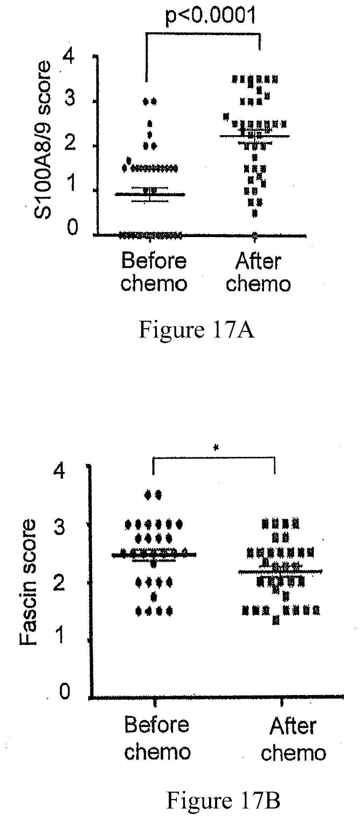

Figure 17A shows S100A8/A9 expression score in paired patient tumor samples, before and after chemotherapy. Data represent expression score. n=40 patients. P values determined by Wilcoxon's paired test, comparing pre and post-treatment levels from each patient.
Figure 17B shows Fascin expression score in paired patient tumor samples, before and after chemotherapy. Data represent expression score. n=32 patients. * represents p=0.01. P values determined by Wilcoxon's paired test, comparing pre and post-treatment levels from each patient.

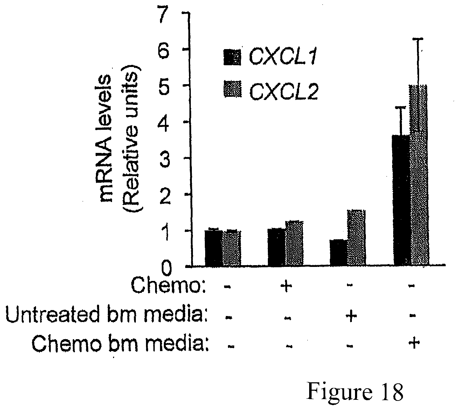

Figure 18 shows *CXCL1*/*2* expression determined by qRT-PCR in LM2 cancer cells either alone, treated with chemotherapy or after incubation with conditioned media from saline treated (bm-media) or doxorubicin treated (chemo bm-media) primary mouse bone marrow derived cells Chemotherapy (chemo): Doxorubicin (0.8µM). Data represent average expression ± SEM.

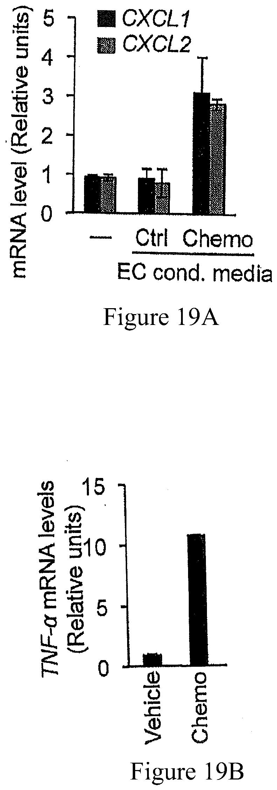

Figure 19A shows *CXCL1*/*2* expression in MDA231-LM2 cancer cells either alone (-) or in the presence of conditioned media from primary human umbilical vein endothelial cells (HUVEC) that were either untreated (*control*) or treated with 0.8µM doxorubicin (*chemo*), as determined by qRTPCR. Data represent average expression ± SEM.
Figure 19B shows TNF-α expression in isolated CD31+ lung endothelial cells from doxorubicin treated tumorbearing mice. n=2-4 mice per group. Data represent averages ± SEM.

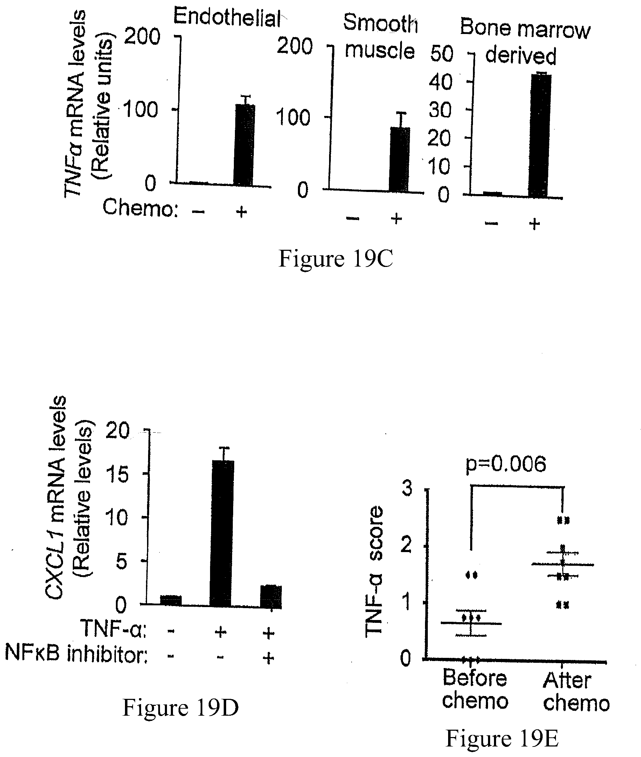

Figure 19C shows *TNF- α* expression in primary endothelial, smooth muscle and bone marrow derived cells treated upon doxorubicin chemotherapy treatment for 16h as determined by qRT-PCR analysis. Error bars represent 95% confidence interval for qRT-PCR analysis. Data is representative of three independent experiments.
Figure 19D shows *CXCL1* expression in LM2 cancer cells treated with vehicle or TNF-α for 2h in the presence of a 100µM NBD (NEMO-binding domain), inhibitory peptide of the NF-κB pathway. Data represent averages ± SEM.
Figure 19E shows a comparison of stromal TNF-α expression score in paired breast tumors before and after chemotherapy. n=8 patients. P value was determined by Wilcoxon's paired test, comparing pre and post-treatment levels from each patient.

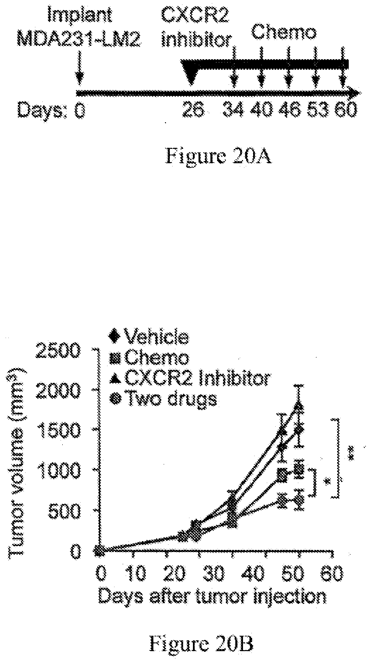

Figure 20A shows a schematic treatment flow.
Figure 20B shows tumor growth f LM2 tumors in mice treated with PEG vehicle or CXCR2 inhibitor for the indicated duration of Figure 20A and subsequent treatment with saline vehicle or AC chemotherapy. Data represent average expression ± SEM. n=10-13 mice per group. P values were determined by Student's t-test. *p=0.02, **p=0.007.

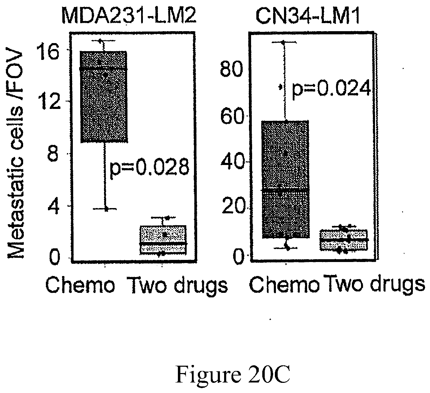

Figure 20C relates to lung metastasis in MDA231-LM2 and CN34-LM1 orthotopic xenograft models undergoing treatment, and shows quantitation of metastasis based on number of cancer cells in lung sections. Data are average foci per field of view (FOV) ± SEM. n=5-10 mice per group. Whiskers represent minimum and maximum values. P values were determined by two-tailed Wilcoxon rank-sum test.
Figure 21 shows relative amount of metastasis as signal from luminescent cells in H2030 cells with and without treatment with shRAGE sequences.
Figure 22 shows relative amount of metastasis as signal from luminescent cells in PC9 BrM cells with and without treatment with shRAGE sequences.

### Detailed Description of the Invention

The present invention makes determinations of the level of expression of S100A8/A9 protein as a prognostic indicator of the therapeutic response to a given type of chemotherapy treatment and as a monitoring indicator of the effectiveness of an on-going chemotherapy treatment for the treatment of breast cancer in human patients.

S100A8 and S100A9 are a pair of low molecular weight, calcium-binding proteins associated with chronic inflammation and upregulated in different types of cancer (Gebhardt et al., 2006; Hobbs et al., 2003). The human mRNA sequence of S100A8 is known from NM_002964.4. This sequence encodes a 93 amino acid peptide having the sequence:

mltelekaln siidvyhkys likgnfhavy rddlkkllet ecpqyirkkg
advwfkeldi ntdgavnfqe flilvikmgv aahkkshees hke Seq ID No. 1

The mRNA sequence of S100A9 is known from NM_002965.3. This sequence encodes a 114 amino acid peptide having the sequence:

mtckmsqler nietiintfh qysvklghpd tlnqgefkel vrkdlqnflk
kenknekvie himedldtna dkqlsfeefi mlmarltwas hekmhegdeg
pghhhkpglg egtp Seq ID No. 2

As used in the present application, the term "S100A8/A9 protein" refers to either of these two proteins individually, or to the two proteins collectively, including in the form of the heterodimer.

In accordance with a first aspect of the invention, a method is provided for treating a human patient suffering from cancer, such as breast or lung cancer by evaluating a patient sample for the amount of S100A8/A9 protein. In some embodiments, the patient sample is a sample of tumor cells, a sample of the surrounding stroma, or a combination thereof. As used in the present application, the term "tumor tissue" refers to any of these three options. The sample may also be a serum sample.

As used in the application, the term "breast cancer" includes localized breast cancers and metastatic cancers believed to have breast cancer origin. Thus, the sample in this case may be taken from a portion of the patient other than breast tissue where breast cancer metastasis is understood to have occurred.

As used in the application, the term "lung cancer" includes localized lung cancer and metastatic cancers believed to have lung cancer origin. Thus, the sample in this case may be taken from a portion of the patient other than lung tissue where lung cancer metastasis is understood to have occurred. In specific embodiments, the lung cancer is non-small cell lung cancer (NSCLC) for example NSCLC that has metastasized to brain or bone.

As used in the specification and claims of this application, the term "assessing responsiveness to chemotherapy treatments" refers to a determination as to whether a patient is likely to be responsive or unresponsive to a selected therapy. Thus, in a first case where the amount of S100A8/A9 protein determined is "low," indicating that the paracrine survival axis has not been activated to confer resistance to conventional standard of care chemotherapy, the conclusion can be reached that standard of care treatment modalities are likely to be effective. In contrast, where the amount of S100A8/A9 protein determined is "high," indicating that the paracrine survival axis has been activated to confer resistance to conventional standard of care chemotherapy, the conclusion can be reached that standard of care treatment modalities are not likely to be effective and/or that therapeutics targeting CXLC1/2 are likely to be effective. Based on this assessment, a course of treatment for the individual patient is selected for example by a physician receiving the test results and the treatment is administered in a conventional manner for the particular treatment.

As used in the this application, the term "standard of care chemotherapy agent" refers to chemotherapy agents used in the treatment of breast cancer, that do not target the TNFα-CXCL1/2-S100A8/A9 paracrine survival axis. By way of example, this includes doxorubicin (aka Adriamycin), cyclophosphamide, and taxanes such a paclitaxel and docitaxel.

In contrast, chemotherapeutic agents that target the TNFα-CXCL1/2-S100A8/A9 paracrine survival axis, includes in particular therapeutics that target the chemokines CXCL1/2/3/8 or their receptors CXCR1/2, the cytokine TNF-alpha or its receptor TNFR, or the factors S100A8/A9 or their receptors RAGE and TLR4. Specific examples of such therapeutics include reperixin ((*R*(-)-2-(4-isobutylphenyl)propionyl methansulphonamide) with a pharmaceutically acceptable counterion); and *N*-(2-hydroxy-4-nitrophenyl)-*N'*-phenylurea and *N*-(2-hydroxy-4-nitrophenyl)-*N*'-(2-bromophenyl)urea, and CXCR1/2 chemokine antagonists as described in WO2005/113534. 4-[(1R)-2-amino-1-methyl-2-oxoethyl]phenyl trifluoromethane sulfonate),has been reported to inhibit both CXCL8- and CXCL1-mediated

PMN chemotaxis with similar potencies. Other compounds are shown in Table 1, reproduced from Chapman et al. Pharmacology & Therapeutics 121 (2009) 55-68 and in US Patent Publication No. 2009/0041753. siRNA inhibitors of CXCL2 are available from SelleckChem (Catalog No. R002920). Kits are also available with siRNA for CXCL1 (Catalog No. R002919). Short hairpin RNA (shRNA) inhibitors of RAGE may also be employed.

Inhibitors of TNF-alpha and its receptor include
infliximab (Remicade), adalimumab (Humira), certolizumab pegol (Cimzia), and golimumab (Simponi), or with a circulating receptor fusion protein such as etanercept (Enbrel). Antibodies, including single chain antibodies targeting the TNF receptor are also known.

Antibodies for inhibition of S100A8/A9 are disclosed in US Patent No. 7553488. Other inhibitors of S100A8/A9 are described in United States Patent Application 20070231317 and include an antibody, preferably a monoclonal antibody capable of binding the S100 protein without affecting other target in the treated organism. Other approaches, such as peptide inhibitors, drugs, anti-mRNA, siRNA, RNAi, transcription or translation inhibitors, can be used as well to perform the method of the present invention which consists in inhibiting or blocking the production or the activity of S100 proteins, and therefore the differentiation or development of progenitor blood cells into leukocytes having cancer behavior. US Patent Publication No. 2010/0166775 discloses methods for identifying inhibitors which block the interaction of S100A9 with RAGE and identifies specific antibodies and a "compound A" (from US 6,077,851) which are effective for this purpose. Small molecule rage inhibitors are shown in table 2, reproduced from Deane et al. (2012), J Clin Invest. 2012; doi:10.1172/JCI58642.

TLR4 inhibitors are available commercially, and include (6R)-6-[[(2-Chloro-4-fluorophenyl)amino]sulfonyl]-1-cyclohexene-1-carboxylic acid ethyl ester (CLI-09, InvivoGen), oxidized 1-palmitoyl-2-arachidonyl-snglycero-3-phosphorylcholine (OxPAPC, InvivoGen) and Ethyl (6R)-6-[N-(2-chloro-4-fluorophenyl)sulfamoyl] cyclohex-1-ene-1-carboxylate (TAK-242, Sha et al. Eur J Pharmacol. 2007 Oct 1;571 (2-3):231-9

The determination of what constitutes a low result versus a high result is dependent on several factors and no specific numeric value is reasonably specified for generic purposes. In general, the value determined is compared to a standard value representing an average amount of S100A8/A9 detected in a comparable sample from an individual who is responsive to conventional chemotherapy using the same methodology. This standard value is referred to in this application as a "relevant" standard value to reflect that the value is determined using the same type of test on the same type of sample. The transition from a low value to a high value will occur at some number greater than the average value, which will depend on two factors: the variability observed in the measurement used to arrive at the average, and the level of confidence desired in the conclusion. For example, the cut off between low and high values may appropriately be set at 1 standard deviation, 2 standard deviations or three standard deviations greater than the average, or some other amount greater than the average selected to separate most patients correctly into one of two groups: those who have an activated TNFα-CXCL1/2-S100A8/A9 paracrine survival axis (high S100A8/A9) and those who do not. A lower cutoff value may be appropriate when dealing with patients known to have refractory disease to one or more standard of care treatments, or to patients with advanced disease when first diagnosed.

In accordance with this method, a sample from the patient is evaluated for the amount of S100A8/A9 protein. Samples may suitably be samples of tumor tissue, for example aspirated or incised biopsy samples. Serum samples may also be used. The evaluation may be performed at the protein level or at the mRNA level, and the method used for the determination is not critical. Hermani et al. 2005, Clin. Cancer Res. 11, 5146-5152, which is incorporated herein by reference, disclose the detection of S100A8 and S100A9 as markers in human prostate cancer using immunohistochemistry to detect proteins, and *in situ* hybridization to detect mRNA for both proteins in tumor tissue, and ELISA to detect the S100A9 protein in patient serum. The S8/S9 heterodimer may also be detected in serum by protein or nucleotide-detection methods, as described in Aochi et al, (2011) J. Amer. Acad Dermatology 64: 869-887, which is incorporated herein by reference. These methods may all be used individually or in combination in the present invention.

Thus, in some embodiments of the method for assessing responsiveness to chemotherapy treatments in a human patient suffering from breast cancer, the evaluation of the patient sample is performed using antibodies that bind to S100A8/A9 protein. Biocompare ® indicates that 49 antibodies to human S100A8 and 7 human antibodies to human S100A9 are commercially available. These antibodies are suitable for use in immunoassays of various types including without limitation immunohistochemistry, ELISA, and Western Blot techniques.

In some embodiments of the method for assessing responsiveness to chemotherapy treatments in a human patient suffering from breast cancer, the evaluation of the patient sample is performed using oligonucleotide probes complementary to the sequences encoding S100A8/A9 as primers (for amplification if desired) and probes. SA Biosciences (Qiagen) sells an RT² qPCR Primer Assay for Human S100A8 as product number PPH19755A.

In some embodiments, a combination of assays detecting protein and nucleic acids are employed. For example, S100A8 may be detected using a protein-detecting assay, while S100A9 is detected with a nucleic acid detecting assay, or vice versa.

In some embodiments of the method of assessing responsiveness, the evaluation for the amount of S100A8/A9 protein is combined with an additional assay to determine the amount of CXCL 1/2 in a sample from the patient. This assay may be of the same type of as the assay for S100A8/A9 protein or different, and may be performed on the same sample or a different sample from the patient.

Additional assays and reagents of S100A8/A9 are disclosed in US Patent Publication No. 2008/0268435, which is incorporated herein by reference. In that publication it is observed that S100 proteins may serve as markers for predicting the presence of non-functional BRCA1 genes, that may lead to a higher risk of breast cancer.

CXCL 1/2 may also be detected using either nucleotide-based or antibody based assays, and there are numerous known reagents useful for each purpose. For example, test kits for determination of human CXCL1 by ELISA are available commercially from MyBiosource (Cat No. MBS494027-IJ27139) and numerous alternative antibodies for ELISA and other immunoassays are known.

Nucleic Acid based assays can be made based on the known sequences of CXCL1 (NM_001511) and CXCL2 (NM_002089) and kits for this purpose are available commercially. (for example, RT² qPCR Primer Assay for Human CXCL1: PPH00696B; RT² qPCR Primer Assay for Human CXCL2: PPH00552E).

In some embodiments of the method of assessing responsiveness, the evaluation for the amount of S100A8/A9 protein is combined with an additional assay to determine the amount of TNF-alpha in a sample from the patient. This assay may be of the same type of as the assay for S100A8/A9 protein or different, and may be performed on the same sample or a different sample from the patient.

TNF-alpha may also be detected using either nucleotide-based or antibody based assays, and there are numerous known reagents useful for each purpose. Kits for detection of human TNF-alpha by ELISA and other immunochemical techniques such as EIA are commercially available, for example from Perkin Elmer (Kit No. AL208C) and numerous other sources. Nucleic Acid based assays can be made based on the known sequences of TNF (human TNF- NM_000594) and kits for this purpose are commercially available (for example RT² qPCR Primer Assay for Human TNF: PPH00341E).

In some embodiments of the method of assessing responsiveness, the evaluation for the amount of S100A8/A9 protein is combined with additional assays to determine the amount of CXCL 1/2 and TNF-alpha in a sample from the patient. These assay may be of the same type (protein or nucleotide detection) as the assay for S100A8/A9 protein or different, and may be of the same type of assay or of different type from one another. These two assays may be performed on the same sample or a different sample from the patient from each other and from the assay for the amount of S100A8/A9 protein.

### Discussion

The major impediments to cure advanced breast cancer are the emergence of pan-resistance to all known chemotherapy drugs and the development of widely metastatic disease, two phenomena that are closely linked clinically (Gonzalez-Angulo et al., 2007). In addressing this challenge, the experimental results set forth below link CXCL1/2 and S100A8/A9 as functional partners of a paracrine loop between breast cancer cells and CD11b+Gr1 +myeloid cells that supports the survival of cancer cells facing the rigors of invading new microenvironments or the impact of chemotherapy (Figure 1). From a therapeutic standpoint, targeting common mediators of chemoresistance and distant relapse would be of interest because these are the two main challenges that patients encounter after primary tumor resection.

The critical role of the microenvironment in tumor progression and response to therapy is being increasingly recognized (Condeelis and Pollard, 2006; DeNardo, 2011; Gilbert and Hemann, 2010; Hanahan and Weinberg, 2011; Qian et al., 2011; Shree et al., 2011; Tan et al., 2011). (Grivennikov et al., 2010). However, the present work sheds light on the more obscure question of how the tumor microenvironment responds to chemotherapy to benefit cancer cell survival. Our evidence from animal models and clinical samples indicate that chemotherapy induces a burst of cytokines including TNF-α from several components of the tumor microenvironment such as endothelial and smooth muscle cells. An undesirable consequence of the stromal TNF-α is to boost CXCL1/2 expression in breast cancer cells. A higher level of CXCL1/2 then drives the paracrine loop involving myeloid cell-derived S100A8/A9 to enhance cancer cell survival (Figure 1). An adverse cycle involving TNF-α-CXCL1/2-S100A8/A9 can thus be expanded in response to chemotherapy. Once initiated, this chemo-protective program could become selfsustaining, leading to the enrichment of residual aggressive clones able to resist chemotherapy and thrive in the lung parenchyma and elsewhere.

### Biological and Clinical Implications

Several additional insights emerge from this work. Regarding CD11b+Gr1 + cells, which are a heterogeneous group of immature myeloid cells (Ostrand-Rosenberg and Sinha, 2009), two roles had been previously discerned for this group of cells in the tumor stroma, namely, angiogenesis and T cell immuno-suppression (Gabrilovich and Nagaraj, 2009; Ostrand- Rosenberg and Sinha, 2009; Shojaei et al., 2007). Our study delineates a new role of CD11+Gr1+ cells in mediating tumor cell survival through the production of S100A8/A9. Given the close link between myeloid cells and adaptive immunity (DeNardo et al., 2010; Ostrand- Rosenberg, 2010), it will be worth exploring how changes in the lymphocyte subsets of CXCL1/2 depleted tumors influence tumor progression. The multi-functional cytokines S100A8/A9 were known to activate MAPK pathways (Gebhardt et al., 2006; Ghavami et al., 2008; Hermani et al., 2006; Ichikawa et al., 2011), which is consistent with our findings in metastatic breast cancer cells. However, we find that S100A8/A9 additionally activate p70S6K as contributors to the pro-survival effect of S100A8/A9 in these cells. In line with our findings, recent Phase 2 study in breast cancer patients showed that non-responders of neoadjuvant chemotherapy and patients with residual disease had significantly higher circulating myeloid-derived suppressor cells (MDSC) levels than did responders (Montero et al., 2011). These findings accentuate the clinical relevance of CD11b+Gr1 + in rendering chemotherapy ineffective and promoting metastasis.

Our findings further indicate that although therapy-induced inflammation is a predominant feature of the use of chemotherapy, disrupting the CXCL1 driven paracrine axis improves therapeutic response in existing lesions and also suppresses metastasis, even at an advanced stage of tumor progression. CXCR2 receptor antagonists are in clinical trials for chronic inflammatory diseases (Horuk, 2009), and these agents are a promising pharmacological approach in metastatic breast cancer when combined with standard chemotherapeutic regimens. The effective combination of chemotherapy with CXCR2 inhibitors at the metastatic site in our preclinical models underscores the potential application of this therapy to limit disseminated tumor burden. Moreover, the important role of CXCR2 in pancreatic adenocarcinoma models (Ijichi et al., 2011) and of S100A8/A9 in colorectal cancer (Ichikawa et al., 2011) indicate that the relevance of targeting the CXCL1/2-S100A8/A9 axis may extend beyond breast cancer.

In conclusion, our results provide mechanistic insights into the link between two major hurdles in treating breast cancer: chemoresistance and metastasis. Our findings functionally unify three important inflammatory modulators, TNF-α, CXCL1/2 and S100A8/A9, in a tumor-stroma paracrine axis that provides a survival advantage to metastatic cells in stressed primary and metastatic microenvironments. This provides the opportunity to clinically target this axis both to limit the dissemination of cancer cells and to diminish of drug resistance.

### Experimental Support

The following experiments provide experimental evidence for the utility of the invention as described in this application.

### CXCL1/2 gene amplification and increased expression in breast cancer

*CXCL1* emerged among a set of genes whose expression is associated with lung relapse in breast tumors, including breast tumors that had not been exposed to prior chemotherapy (Minn et al., 2007; Minn et al., 2005) and as a gene that enriches the aggressiveness of seeded primary tumors (Kim et al., 2009). From gene expression analysis of a combined cohort of 615 primary breast cancers, we found that the two CXC chemokines, *CXCL1* and *CXCL2* showed very similar expression profile. *CXCL1* and 2 are 90% identical by sequence and signal through the same CXCR2 receptor (Balkwill, 2004), however their role in breast cancer progression and metastasis remains elusive. Recent genome-wide gene copy number analysis revealed copy number alterations at 4q21 (chr 4:73526461-75252649) in breast cancer (Beroukhim et al., 2010). The fifteen genes in the amplification peak include several members of the CXC family of chemokines including *CXCL1-8.* However, in a separate study (Bieche et al., 2007), high expression of *CXCL1* and related CXC chemokines in breast tumors was attributed to transcriptional regulation with no evidence of amplification. These results prompted us to specifically analyze *CXCL1*/*2* amplification in breast cancers and metastases. Fluorescence-in-situ hybridization (FISH) analysis using probes specific for *CXCL1*/*2* showed that *CXCL1* and *CXCL2* genes were amplified in approximately 8% of primary breast tumors and in 17% of lymph node metastases (Figure 2, Tables 3 and 4). These results suggested that increased copy number contributes in part to the higher *CXCL1*/*2* expression in invasive breast cancers and metastases. Collectively these findings provided us with a rationale to explore a potential role of *CXCL1*/*2* in breast cancer progression and particularly metastatic recurrence to the lungs.

### CXCL1/2 mediate tumor growth and lung metastasis

To evaluate the functional role of CXCL1 and 2 in breast cancer progression and metastasis, we utilized two different systems. First, a syngeneic transplant system with primary tumor cells referred to as PyMT cells, that we derived from the MMTV-PyMT mouse model of mammary cancer driven by a polyoma middle T transgene (Lin et al., 2003). Second, a xenograft model to implant LM2 lung metastatic cells that were derived from the MDA-MB-231 human breast cancer cell line (Minn et al., 2005). Consistent with our clinical evidence, LM2 lung metastatic cells showed significant upregulation of *CXCL1*/*2* compared to the parental lines (Figure 3). Both cell lines grew aggressively in the mammary fat pad and readily metastasized to the lungs. We stably reduced the expression levels of *CXCL1* and 2 using short hairpin RNA interference in PyMT and LM2 cells.

Knockdown of *CXCL1* and 2 using two independent hairpins (Figures 4A-4C) significantly reduced tumor growth upon inoculation into the mammary fat pad (Figures 5A-F). Decreased mammary tumor growth in both models was associated with reduced metastasis in the lungs. A similar trend was observed upon size-matching knockdown tumors to controls (Figures 6A and B). A lung colonization assay by tail vein injection of LM2 cells confirmed that CXCL1/2 mediates lung metastasis and the defect of CXCL1/2 knockdown cells is not solely a consequence of decreased tumor burden (Figures 7A and B). Together, these results suggest that CXCL1/2 enhance breast cancer progression and metastasis.

### CXCL1/2 chemokines recruit myeloid cells for cancer cell survival

Reduction in CXCL1/2 levels in both the LM2 xenograft and MMTV-PyMT transplantation model was associated with a significant increase in apoptosis in the tumors (Figures 8A and B). However, the effects of CXCL1/2 on survival were not accompanied by any visible changes in angiogenesis or cell proliferation rates (Figures 9A-D). The function of CXCL1/2 is primarily mediated by binding to the G-protein coupled receptor, CXCR2 and in some instances CXCR1 and DARC (Balkwill, 2004; Raman et al., 2007). Compared to the appreciably high levels of the CXCL1/2 ligands in the lung metastatic cell-lines, CXCR1, CXCR2 and DARC receptor expression was negligibly low both at the RNA and protein levels (see, Muller et al., 2001). Based on these results, we explored the possibility that CXCL1/2 could mediate tumor cell survival via paracrine mechanisms.

CXCR1 and 2 are expressed by several stromal cell types such as endothelial cells, cells of myeloid origin and a subset of T cells (Murdoch et al., 2008; Stillie et al., 2009). We did a comprehensive analysis of major cell types in the tumor microenvironment whose abundance changed upon *CXCL1*/*2* knockdown in both the LM2 xenograft and PyMT transplant model. A striking reduction in CD11b+Gr1+ myeloid precursors and neutrophils was observed in *CXCL1*/*2* knockdown tumors in both models plus a decrease in CD68+ macrophages in the LM2 model (Figures 10A and B). Myeloid precursor cells represent a heterogeneous group of immature myeloid cells including precursors for neutrophils and monocytes (Joyce and Pollard, 2009; Murdoch et al., 2008; Shojaei et al., 2007). In contrast, no detectable differences were observed in myofibroblast, erythroid, endothelial, B or T cell numbers in the tumors. We concluded that CD11b+Gr1 + myeloid cells represent predominant cell types recruited by CXCL1/2 in the tumor microenvironment in metastatic breast cancer models.

### CXCL1/2 mediates its paracrine survival function through myeloid S100A8/A9

Results from our functional analysis suggested to us that myeloid cell types recruited by CXCL1/2 release paracrine factors that can provide tumor cells with survival advantage. To determine the identity of these stromal factors, we analyzed gene expression datasets from breast cancer patients for genes that are expressed in association with *CXCL1* (Figure 11A). Focusing on paracrine mediators, we filtered genes encoding cell surface and secretory products. Analysis of 615 breast tumors from three independent datasets yielded a list of 43 such genes that correlated with *CXCL1* with a correlation coefficient >0.3 (Figure 11A and Table 5). Top *CXCL1* correlating genes showed a predominance of chemokines (40%) and cytokines (21%) (Table S3). These genes included the cytokines *IL6, TNF-α* and *IFN*γ, some of which can induce *CXCL1* transcription (Amiri and Richmond, 2003) and have been implicated in cancer progression (Grivennikov et al., 2009; Kim et al., 2009), and chemokines implicated in metastatic progression such as *CCL2* (Nam et al., 2006), *CCL5* (Karnoub et al., 2007), *CXCL5* (Yang et al., 2008), *CXCL8*/*IL8* (Kim et al., 2009) and *S100A8*/*A9* (Hiratsuka et al., 2008). To identify mediators of a CXCL1/2 paracrine loop, we experimentally interrogated genes encoding extracellular proteins whose expression most significantly correlate with *CXCL1* (Figure 11A and Table 5). We searched for candidates that are abundantly expressed in myeloid cell types recruited by CXCL1/2 and are not of epithelial origin (Figure 11B, Table 5 and 6). Based on these criteria, *S100A8*/*A9* genes were identified.

S100A8/A9 bind to cell surface receptors TLR4 and RAGE (receptor for advanced glycation end products), which are multi-ligand receptors that initiate signaling cascades activating multiple downstream pathways such as NFκB, PI3K, MAPK and Stat3 (Gebhardt et al., 2006; Turovskaya et al., 2008). Both TLR4 and RAGE are expressed in breast cancer cells (Bos et al., 2009; Ghavami et al., 2008; Hsieh et al., 2003) and therefore could be involved in S100A8/A9 function. To determine whether myeloid S100A8/A9 can mediate survival of metastatic breast cancer cells, we isolated primary bone marrow derived-CD11 b+Gr1 + cells from *S100a9*+/+ and *S100a9-*/*-* mice (Hobbs et al., 2003). In addition to lacking *S100a9,* bone marrow derived cells from *S100a9-*/*-* mice fail to express S100a8 protein, which is the heterodimeric partner of S100a9 (Hobbs et al., 2003). Consistent with the survival advantage provided by bone marrow derived factors (Joyce and Pollard, 2009), co-culture of tumor cells with *S100A9+*/*+* primary CD11b+Gr1 + myeloid precursor cells protected tumor cells from doxorubicin-induced apoptosis (Figure 12A). However, this protection was partially lost in tumor co-culture with myeloid precursor cells lacking S100A8/A9 (Figure 12A), indicating that the pro-survival properties of myeloid factors under stressed conditions can in part be attributed to S100A8/A9.

Based on the survival functions imparted by S100A8/A9 in vitro, we asked whether S100A8/A9 enhances tumor growth and metastasis in vivo. We isolated bone marrow cells from *S100A9*+/+ and *S100A9*-/- mice and transplanted into irradiated immunocompromised mice lacking B,T and NK cells. After confirming successful engraftment of *S100A9*+/+ or *S100A9-*/*-* bone marrow with an efficiency of >98%, we implanted LM2 cancer cells in the mammary fat pads of these mice. Mammary tumor growth and lung metastasis were significantly slower in mice transplanted with *S100A9-*/*-* bone marrow compared to the *S100A9*+/+ counterpart (Figure 12B-C). Consistent with our hypothesis, tumors growing in mice transplanted with *S100A9-*/*-* bone marrow exhibited increased apoptosis.

In the light of these results, we asked whether *S100A8*/*A9* expression in cancer cells could "short circuit" and rescue the CXCL1/2 knockdown phenotype of reduced tumor growth and metastasis. Since LM2 cancer cells do not express any appreciable levels of S100A8/A9, we overexpressed S100A8/A9 in CXCL1/2 knockdown tumor cells. In line with our hypothesis, S100A8/A9 expression phenotypically rescued CXCL1/2 deficiency by restoring both tumor growth and lung metastasis (Figure 13). Together, these results indicate that S100A8/A9 mediates the metastatic functions of CXCL1/2.

Based on the accumulating evidence supporting an important function of S100A8/A9 in breast cancer metastasis in animal models, we sought clinical evidence for a link between S100A8/A9 and lung metastasis. We immunostained tissue microarrays composed of lung metastasis samples from breast cancer patients with an antibody recognizing human S100A9 protein. Kaplan Meier analysis showed that patients with high S100A9 in the metastatic nodules had a significantly shorter overall survival compared to low S100A9 (p-value=0.01) (Figure 14). Collectively our functional studies suggest that CXCL1/2 function in breast cancer cells is mediated by stromal S100A8/A9, which in turn provides a critical survival advantage that promotes breast cancer metastasis.

### CXCL1/2-S100A8/A9 survival axis is hyperactivated by chemotherapy

Most patients who develop metastatic disease receive chemotherapy at some point in the management of their illness. Tumor shrinkage-partial and less commonly complete remissions-is usually accomplished, but these benefits are transient and most patients eventually die of chemotherapy-resistant and widely disseminated cancer (Gonzalez-Angulo et al., 2007; Jones, 2008). We hypothesized that the CXCL1/2-S100A8/A9 survival axis could nurture tumor cells under chemotherapeutic stress thereby selecting for aggressive metastatic progeny. To address this question, we treated mice bearing LM2 tumors with doxorubicin (Adriamycin®) and cyclophosphamide (AC), a commonly used chemotherapy combination in the clinic. MDA-MB-231, the parental breast cancer cell line from which LM2 was derived, was originally isolated from pleural effusion of a patient who was resistant to 5-fluorouracil, doxorubicin and cyclophosphamide chemotherapy and had relapsed (Cailleau et al., 1974).

Chemotherapy treatment in the mice initially resulted in significant apoptosis and a concomitant delay in tumor growth (Figures 15A-B). However, after subsequent rounds of chemotherapy, these aggressive cancer cells showed refractoriness to therapy as evidenced by significant reduction in apoptosis and resumed tumor growth (Figure 15A and B). We wanted to determine whether the CXCL1 mediated paracrine interactions was a mediator of increased cancer cell survival during chemotherapy challenge. To address this question, we analyzed the expression of *CXCL1* and *CXCL2* in AC treated tumors. Indeed, quantitative RT-PCR analysis of whole tumors showed that AC chemotherapy treated tumors significantly upregulated *CXCL1*/*2* (Figure 15C). Consistent with our results, higher *CXCL1*/*2* induction was associated with increased recruitment of S100A8/A9 expressing myeloid cells (Figure 15D). CXCL1/2 upregulation was not restricted to AC chemotherapy regimen but was also observed with another commonly used chemotherapeutic drug, paclitaxel in the LM2 tumors (Fig. 16A). In addition to *CXCL1*/*2* and *S10OA8lA9,* other *CXCL1*-associated chemokine genes such as *CCL20* and *CXCL3* were also induced upon chemotherapy treatment (Figures 16B and C). These results suggest that chemotherapy activates a "burst" of chemokines, of which we show S100A8/A9 promote cancer cell viability and select for clones that avert chemotherapy.

### S100A8/A9 association with resistance to perioperative chemotherapy

Neoadjuvant chemotherapy-the use of cytotoxic drugs prior to surgery for primary breast cancer-is an option for patients with operable disease. This has long been the standard approach for patients with locally advanced, inoperable primary disease in an effort to shrink the tumor and thereby make complete tumor removal possible. While these treatments usually cause tumor volume regression, some cases are chemotherapy-resistant *de novo* (Gonzalez-Angulo et al., 2007). To address whether the CXCL1/2-S100A8/A9 survival loop is activated in cancer patients with primary disease, we stained matched breast tumor sections from a cohort of patients before and after chemotherapy treatment. Based on the chemo-protective functions of S100A8/A9, we asked whether the number of S100A8/A9-expressing cells increases after neoadjuvant chemotherapy treatment and whether this is linked to therapy response. Indeed consistent with our experimental models, a significant increase in S100A9 expressing cells was observed in breast cancers after chemotherapy treatment (Figure 17A). In contrast, Fascin that is a part of a lung metastasis signature (Minn et al., 2005) did not show the same trend upon chemotherapy treatment. (Figure 17B) Furthermore, comparison of pathological response in chemotherapy treated patients showed 11/12 and 9/9 of the partial and minimal responders, respectively showed an increase in the number of S100A9 expressing cells when compared to pretreatment. However, only 1 out of 4 of the complete responders showed a modest increase in S100A9 positive myeloid cell numbers after treatment. This suggests that chemotherapy induces recruitment of S100A8/A9 expressing cells that might promote resistance to the treatment by providing a protective environment for residual tumor cells.

### TNF-α from chemotherapy-activated stroma enhances the CXCL1/2-S100A8/A9 axis

Hyperactivation of the CXCL-S100A8/A9 loop upon chemotherapy treatment prompted us to explore the mechanism behind therapy induced CXCL1/2 upregulation. However in our experimental models, enhanced expression of CXCL1/2 in response to chemotherapy was not due to additional amplification of the locus as determined by FISH analysis. Being target genes of NF-κB and Stat1 pathways (Amiri and Richmond, 2003), we reasoned that *CXCL1*/*2* upregulation in response to chemotherapy could instead be mediated by activation of these inflammatory pathways directly by the treatment. This was ruled out because treatment of LM2 cells with chemotherapeutic agents did not induce CXCL1/2 expression (Figure 18). However LM2 tumor cells incubated with conditioned media from chemotherapy-treated, but not from untreated endothelial cells, showed a significant increase in CXCL1/2 expression (Figure 19). This effect was not restricted to endothelial cells as treatment with conditioned media from bone marrow-derived cells also induced *CXCL1*/*2* expression in tumor cells.

To identify factors expressed by cells in the stroma that induce *CXCL1*/*2* transcription in cancer cells, we examined a panel of prototypical inducers of the NF-κB/Stat1 pathway. Quantitative RT-PCR analysis showed that *TNF-α* was strikingly induced in endothelial and bone marrow cells upon chemotherapy treatment in-vitro. Consistent with our in vitro results, quantitative PCR analysis showed a ten-fold induction of *TNF-α* in purified lung endothelial cells from LM2 tumor bearing mice systemically treated with AC chemotherapy (Figure 20A). NF-κB activation via TNF-α can mediate *CXCL1*/*2* transcription (Amiri and Richmond, 2003), which was the case in LM2 tumor cells (Figure 19C). Pharmacological inhibition of the NF-KB pathway selectively resulted in a reduction in tumor derived *CXCL1*/*2* expression in the presence of TNF-α (Figure 19D). Thus, TNF-α from chemotherapy-activated stroma can induce and sustain the CXCL1/2-S100A8/A9 loop.

To examine whether TNF-α induction from chemotherapy treated stroma also occurred in breast cancer patients, we immunostained tumors from patients with primary disease before and after preoperative chemotherapy with an antibody against TNF-α. Consistent with our findings in breast cancer models, significant increase in TNF-α staining was observed in patient samples after neoadjuvant AC chemotherapy treatment (Figure 19E). Importantly, histopathological analysis revealed that cells from the tumor microenvironment specifically lymphatic and blood vessels and fibroblast-rich stromal areas showed strong TNF-α staining particularly after chemotherapy. Collectively, TNF-α spike induced by chemotherapy reinforces the CXCL1/2-S100A8/A9 survival axis in aggressive metastatic progenies.

### Targeting the CXCL1-driven paracrine axis enhances chemotherapy response in metastatic breast cancer

Our findings indicate that a self-defeating consequence of the administration of at least some chemotherapy drugs is the release of potent pro-inflammatory cytokines such as TNF-α from stromal sources. Such pro-inflammatory bursts can fuel the CXCL1/2-S100A8/A9 survival axis and facilitate the selection and maintenance of aggressively metastatic clones. These results presented us with two general options of targeting the tumor microenvironment in an attempt to sensitize breast cancer cells to chemotherapy: (1) targeting the pro-inflammatory cytokine burst that is amplified upon chemotherapy or (2) targeting the CXCL-CXCR2 axis that is pivotal for myeloid recruitment into the tumor microenvironment. Because of the modest responses activity despite considerable toxicity observed upon systemic inhibition of pro-inflammatory cytokines (Balkwill, 2009; Baud and Karin, 2009), we decided against the first option. Instead we utilized antagonists of CXCR2, the primary receptor for CXCL1/2, since derivatives of these pharmacological inhibitors are in clinical trials for chronic inflammatory diseases and show no major toxicity issues with long-term usage (Busch-Petersen, 2006; Chapman et al., 2009). Furthermore, targeting the immune microenvironment might be an attractive option because of the potentially low selective pressure for mutations and epigenetic changes on the stroma compared to the tumor genome. Based on this rationale, we designed preclinical trials in mice with a combination of AC chemotherapy and CXCR2 antagonist in two aggressive, lung metastatic human breast cancer cell lines, LM2 and a more recently derived pleural effusion isolate, CN34LM1 from a stage IV breast cancer patient (Tavazoie et al., 2008) (Fig. 20A). Tumor-bearing mice treated with AC chemotherapy alone showed a reduction in tumor growth (Figure 20B). However metastatic cells were not completely eliminated and micrometastases were detected throughout the lungs. Importantly, when AC was combined with the CXCR2 inhibitor, the lung metastatic burden was markedly reduced. (Figure 20C) Immunostaining showed a significant reduction in S100A9 expressing cells in the CXCR2 inhibitor/chemotherapy treatment despite TNF levels remaining high. These findings suggest that although therapy induced inflammation is a predominant feature of the use of chemotherapy, disrupting the CXCL1 driven paracrine axis was sufficient to both improve therapeutic response in existing lesions and also inhibit lung metastasis, even at an advanced stage of tumor progression.

### Knockdown of RAGE reduces brain and bone metastasis in NSCLC

Two luciferase-labeled metastatic lung cancer cell lines (PC9 and H2030) wereinjected into the arterial circulation of athymic mice, and brain and bone metastasis was monitored over time by bioluminesnece imaging. Knockdown of RAGE was accomplished using shRNA. Different sequences were found to be more effective in the different cells lines. Thus, shRAGE#5 and shRAGE#6 had the greatest effect in H2030 cells, while shRAGE#1 and shRAGE#2 had the greatest effect in PC9 cells as determined by qRT-PCR .

Knockdown of RAGE was observed to reduce brain and bone metastasis of the NSCLC cell lines. In particular, in H2030 treatment with shRAGE#5 or shRAGE#6 reduced observed photon flux from the luminescent cell lines by about one or more orders of magnitude 4 weeks after injection. (Figure 21) In mice injected with PC9 cells, treatment with shRAGE#1 (but not shRAGE#2) resulted in a reduction in photon flux of about 2 orders of magnitude. (Figure 22). Without intending to be bound by any particular mechanism, it is possible that the lack of effect on metastasis of shRDA#2 was the result of the failure to maintain knockdown *in vivo* after recovering cancer cells.

### EXPERIMENTAL PROCEDURES

**Cell culture and in-vitro treatments.** MDA231-LM2, 293T and PyMT cells were grown in DME media supplemented with 10% fetal bovine serum (FBS), 2mM L-Glutamine, 100IU/mL penicillin, 100µg/mL streptomycin and 1µg/mL amphotericin B. All primary bone marrow derived cells, including purified CD11b+Gr1 + cells, were maintained in RPMI media supplemented with 10% heat inactivated fetal bovine serum (FBS), 2mM L-Glutamine, 100IU/mL penicillin, 100µg/mL streptomycin and 1µg/mL amphotericin B during coculture. The CN34-LM1 cell line was maintained in M199 media containing 2.5% FBS, 10µg/mL insulin, 0.5µg/mL hydrocortisone, 20ng/mL EGF, 100ng/mL cholera toxin, 0.5µg/mL amphotericin B, 2mM LGlutamine, 100IU/mL penicillin and 100µg/mL streptomycin. Retroviral packaging cell line GPG29 was maintained in DME media with 2mM L-Glutamine, 50IU/mL penicillin, 50µg/mL streptomycin, 20ng/mL doxycycline, 2µg/mL puromycin and 0.3mg/mL G418. Primary HUVEC, (human umbilical vein endothelial cells), HBSMC (Human bronchial smooth muscle cells) were purchased from ScienCell, MPRO and U937 were purchased from ATCC and grown following manufacturer's instructions. Recombinant TNF-α and NBD (NEMO binding domain inhibitory peptide) were purchased from Roche and Imgenex, respectively, and reconstituted following manufacturer's instructions. Recombinant human CXCL1, CXCL2 and mouse Cxcl1/KC, Cxcl2/MIP-2 was purchased from R&D Systems. Coculture of cancer and tumor microenvironment cells (bone marrow derived cells, purified myeloid precursors or HUVEC) was done for a period of 12-16h prior to initiating all treatments. Incubation with conditioned media or admixture of cells during treatment was done for 2h and 4h, respectively. For experiments involving recombinant S100A8/A9, MDA231-LM2 cells pretreated with S100A8/A9 (Calprotectin) from Hycult Biotech for 1 hr, were treated with either Doxorubicin (Sigma) at 0.8µM alone or in combination with p38 inhibitor (SB 203580 from Cell Signaling) at 5µM, S6K inhibitor (PF4708671) at 10µM or Erk1/2 inhibitor (FR180204) at 10µM for 16 hrs. Cells were washed with PBS, fixed in 4% PFA for 1 hr and TUNEL assay was performed using In situ Cell death Detection kit, TMR Red (Roche) following manufacturer's instructions.

**Cytogenetics.** FISH analysis for both cells and tissues were performed at the MSKCC Molecular Cytogenetics Core Facility using standard procedures (Gopalan et al., 2009; Leversha, 2001). Probes used in this assay were made from BAC clones RP11-957J23 spanning *CXCL1* locus, and RP11-1103A22 spanning *CXCL2* locus. For FISH on cell lines RP11-957J23 was labeled by nick translation with Green dUTP and RP11-1103A22 was labeled with Red dUTP (Enzo Life Sciences, Inc., supplied by Abbott Molecular Inc.). A chromosome 4q centromeric BAC, RP11-365A22 labeled with Orange dUTP was included for reference. 10 DAPI-banded metaphases and at least 10 interphase nuclei were imaged per sample. For tissue sections, both *CXCL2* BACs were labeled with red-dUTP and the reference probe was augmented with BAC clone RP11-779E21. For image clarity, the orange reference probe was displayed as green. All FISH signals are captured using a monochrome camera and images were pseudocolored for display. For FISH analysis detecting X and Y chromosomes, repetitive probes for mouse X and Y chromosomes were made from plasmid DXWas70 labeled with Red dUTP and BAC clone CT7-590P11 (Y heterochromatin purchased from Invitrogen) labeled with Green dUTP (Abbott Molecular). 500 cells per slide were scored for each sample independently by 2 members of the Molecular Cytogenetics Core Facility.

**Generation of CXCL1/2 knockdown cells and S100A8/A9 rescue cells.** *CXCL1*/*2* genes were knocked down using custom designed retroviral pSuperRetro based constructs or pLKO.1 lentiviral vectors expressing short hairpins targeting the gene products using TRCN0000057940, TRCN0000057873, TRCN0000372017 from Sigma/Open Biosystems. CXCL3 was knocked down using human GIPZ lentiviral shRNAmir target gene set (Open Biosystems) using V2 LHS_223799 and V2 LHS_114275. *CXCL5* and *S100A8*/*A9* were knocked down using pLKO.1 lentiviral vectors expressing shRNA against the gene products using TRCN0000057882, TRCN0000057936, TRCN0000104758, TRCN0000072046, respectively obtained from Open Biosystems. Lentiviral particles were used to infect subconfluent cell cultures overnight in the presence of 8µg/mL polybrene (Sigma-Aldrich). Selection of viral infected cells expressing the shRNA was done using 2µg/mL puromycin (Sigma-Aldrich) in the media. To generate S100A8/A9 rescue cells, *S100a8* and *S100a9* was amplified by PCR from 2 complete cDNA clones from Open Biosystems and ATCC, respectively and subcloned in to pBabe-hygromycin retroviral vector via Eco R1 and Sal1 restriction sites for *S100a8* and BamH1 and Sal1 restriction sites for *S100a9*. PCR primers for *S100a8*: Forward, 5'-CAG AAT TCA TGC CGT AAC TGG A-3' (Seq ID No. 3) and reverse, 5'-CCA GTC GAC CTA CTC CTT GTG GCT GTC TTT GT-3' 9Seq ID No. 4). PCR primers for *S100a9*: Forward, 5'-TAA GGA TCC ATG ACT TGC AAA ATG TCG CAG C-3' (Seq ID No. 5). Reverse, 5'-TAA TGT CGA CTT AGG GGG TGC CCT CCC C-3' 9seq ID No. 6). Retroviral particles were packed using GPG29 packaging cell line transfected with retroviral constructs. Transfection reagent used was Lipofectamine 2000 (Invitrogen). Selection for S100A8/A9 expressing cells was done using 500µg/mL hygromycin (Calbiochem) in media. Knockdown of RAGE was performed using shRNA obtained from Open Biosystems. shRAGE#1: TRCN0000377641; SHRAGE #2: TRCN0000371283 No. 4); shRAGE#5: TRCN0000062661; and shRAGE #6: TRCN0000062660.

**Gene expression analysis.** Whole RNA was isolated from cells using PrepEase RNA spin kit (USB). 100-500ng RNA was used to generate cDNA using Transcriptor First Strand cDNA synthesis kit (Roche). Gene expression was analyzed using Taqman gene expression assays (Applied Biosystems). Assays used for human genes: *CXCL1* (Hs 00236937-m1), *CXCL2* (Hs00236966_m1), *CXCL3* (Hs00171061_m1), *CXCL5* (Hs00171085_m1), *EGFL6* (Hs00170955_m1), *CCL2* (Hs00124140_m1), *CCL18* (Hs00268113_m1), *CCL20* (Hs01011368_m1), *EGFR* (Hs01076078_m1), *IL1β* (Hs01555410_m1), *IL6* (Hs00985639_m1), *TNF-α* (Hs00174128_m1), *TNF*β (Hs00236874_m1), *IL2* (Hs00174114_m1), *GMCSF* (Hs00929873_m1), *IFNα1* (Hs00256882_s1), *IFN*γ (Hs00989291_m1). Assays used for the mouse genes: *Ccl*2 (Mm00441242_m1), *Cc*/*20* (Mm01268754_m1), C*xcl5* (Mm00436451_g1), *Cxcl3* (Mm01701838_m1), *S100a8* (Mm00496696_g1), *S100a9* (Mm00656925_m1), *Egfl6* (Mm00469452_m1), *Egfr* (Mm00433023_m1), *Cxer1* (Mm00731329_s1), *Cxcr2* (Mm00438258_m1). Relative gene expression was normalized to the "housekeeping" genes β*2M* (Hs99999907_m1) and β*-actin* (Mm02619580_g1). Quantitative PCR reaction was performed on ABI 7900HT Fast Real-Time PCR system and analyzed using the software SDS2.2.2 (Applied Biosystems). Statistical analysis was performed using Graphpad Prism 5 software.

**Flow cytometric analysis and Magnetic Separation.** Whole tumors or lung tissues were dissected, cut into small pieces and dissociated using 0.5% collagenase Type III (Worthington Biochemical) and 1% Dispase II (Roche) in PBS for 1-3 h. Resulting single cell suspensions were washed in PBS with 2% heat-inactivated fetal calf-serum and filtered through 70 µm nylon mesh. Eluted cell fractions were incubated for 10 mins at 4°C with anti-mouse Fc block CD16/32 antibody (2.4G2 BD) in PBS containing 1% BSA to avoid non-specific antibody binding. Cells were subsequently washed in PBS/BSA and stained with either Ig controls or fluorophore conjugated antibodies mentioned below in MACS buffer (0.5%BSA, 2 mM EDTA in PBS). Data acquisition was performed on a FACSCalibur (BD Biosciences) or Cytomation CyAn (Beckman Coulter) and analysis was done using Flowjo version 9 (Tree star, Inc.). Antibodies used: Antimouse antibodies from eBioscience were Ly6C Clone HK1.4, CD34 Clone RAM34, CD80 (B7-1) Clone 16-10A1, CD86 (B7-2) Clone GL1, γδ TCR Clone GL3, CD3 Clone 17A2, CD31 Clone 390, CD25 Clone PC61.5, CD8α Clone 53-6.7, CD49b Clone DX5, F4/80 Clone BM8, Antimouse/human CD45R/B220 Clone RA3-6B2; anti-mouse antibodies from R&D Systems were goat polyclonal IL4R□, VEGF R1 Clone 141522; anti-mouse antibodies from BD Biosciences were CD45 Clone 30-F11, Ly6G Clone 1A8, CD4 Clone GK1.5, Sca1 Clone D7, CD117 Clone 2B8; Rat monoclonal antibodies from Miltenyi Biotech were CD11b Clone M1/70.15.11.5 that recognizes both human and mouse CD11b antigen and anti-mouse Gr1 Clone RB6-8C5.

For analysis, CD11b+Gr1 hi cells were isolated by a combination of magnetic purification and FACS sorting from dissociated tumors. Briefly, cells were positively selected using CD11b magnetic microbeads (Miltenyi Biotech), purity and cell number were assessed by flow cytometry using CD11b-APC following manufacturer's instructions. Eluted cell fractions were incubated for 10 mins at 4°C with anti-mouse Fc block CD16/32 antibody (2.4G2 BD) in PBS containing 1%BSA. Cells were subsequently washed in PBS/BSA and stained with either Ig controls or Gr1 (Miltenyi biotech) in MACS buffer (0.5%BSA, 2 mM EDTA in PBS) following manufacturer's instructions. Cells were analyzed by flow cytometry as described before. For lung tissues, single cell suspension was prepared as mentioned above and labeled with either Ig control or CD31 antibody (clone 390) from eBioscience. Cells were sorted using FACS Aria, washed once with PBS, collected in cell lysis buffer (PrepEase Kit, USB) and frozen in -80°C for subsequent RNA isolation. For flow cytometric analysis on blood, mice were bled from the tail and processed as described previously (Sinha et al., 2008). For flow cytometric analysis of CXCR1 and 2 receptors on cancer cells, cells were incubated with mouse monoclonal antibodies against CXCR1 (clone 42705) and CXCR2 (clone 48311) from R&D Systems using manufacturer's recommendations. For fresh isolation of CD11b+Gr1 + cells from bone marrow for tumor coculture, cells were magnetically sorted for CD11b and Ly-6G double positive fractions following manufacturer's instructions (Miltenyi Biotech). In brief, bone marrow cells were labeled with CD11b-PE (Miltenyi Biotech.) and magnetically sorted using Anti-PE multisort microbeads. Positively labeled CD11b+ cells were incubated with multisort release reagent followed by multisort stop reagent. Cells were subsequently labeled with Anti-Ly-6G-biotin and Anti-Biotin microbeads and magnetically labeled CD11b+Ly-6G+ fractions were eluted. Cells were plated in RPMI media supplemented with 10% heat inactivated fetal bovine serum (FBS).

**Morphometric Analysis.** Tumor vessel characteristics and lung metastatic foci size and number were quantified using Metamorph software (Molecular Devices) as previously described (DeNardo et al., 2009; Gupta et al., 2007). In brief, 10 random images at 20X magnification were taken per tumor section stained with CD34, MECA32 or Von Willebrand factor by immunohistochemistry. Images were thresholded, stained area was calculated by counting objects per field and vessel characteristics were analyzed using Metamorph measurement module. For quantitating lung metastatic foci area and number, 8-12 random images at 20X magnification were taken per lung section stained with vimentin by immunohistochemistry. A minimum of 5 sections was analyzed per animal across different depths of the tissue. For quantitation, images were thresholded and number of metastatic foci determined. Metastatic foci was considered if they contained more than 5 cells. Foci were counted and analyzed using Metamorph measurement module.

**Immunofluorescence and TUNEL staining.** Tissues were fixed in 4% paraformaldehyde at 4°C overnight. After PBS washes, tissues were mounted and frozen in OCT compound (VWR) and stored at -80°C. 8 µm thick cryosections were used for TUNEL assays using In situ Cell death Detection kit, TMR Red (Roche) following manufacturer's instructions. For immunostaining, cryosections were incubated with a blocking buffer (Mouse on mouse- MOM kit, Vector Laboratories) followed by overnight incubation with the primary antibody of interest at 4°C in diluent (MOM kit, Vector Laboratories). The following antibodies were used: rat antimouse CD68 (clone FA-11) from AbD Serotec, mouse anti-human alpha smooth muscle actin (clone 1A4) from Dako, rat anti-mouse CD11b (Clone M1/70) from BD Pharmingen. The sections were incubated at room temperature for 30 minutes with the corresponding fluorochrome conjugated secondary antibodies (Molecular Probes). Species matched isotype antibodies were used as negative controls. Slides were mounted in aqueous mounting media containing DAPI (Fluorogel II from Electron Microscopy Sciences). Stained tissue sections were visualized under a Carl Zeiss Axioimager Z1 microscope.

**Histological staining.** Tissues were fixed overnight at 4°C in 4% paraformaldehyde for mouse tissues and in 10% formalin for human tissues, paraffin-embedded and sectioned. 5 µm thick tissue sections were baked at 56°C for 1 h, de-paraffinized and treated with 1% hydrogen peroxide for 10 mins. For staining, antigen retrieval was performed either in citrate buffer (pH 6.0) or in alkaline buffer (pH 9.0) from Vector labs. Sections were incubated with a blocking buffer (MOM kit, Vector Laboratories) followed by primary antibody of interest. Corresponding biotinylated secondary antibodies and ABC avidin-biotin-DAB detection kit (all from Vector laboratories) were used for detection and visualization of staining following manufacturer's instructions. Sections were subsequently counterstained with Hematoxylin and analyzed under Zeiss Axio2Imaging microscope. Vimentin, cleaved caspase 3, CD34, phospho-histone H3, were performed by the MSKCC Molecular Cytology Core Facility using standardized automated protocols. Antibodies: Vimentin (Clone V9) (Vector laboratories), Rabbit polyclonal Cleaved caspase 3 Asp175(Cell Signaling), Rabbit polyclonal Von Willebrand Factor (Millipore), Rat monoclonal MECA-32 (Developmental Hybridoma Bank, Iowa), CD34 clone RAM34 (Ebioscience), phospho-histone H3 Clone 6570 (Upstate), Rat monoclonal TER-119 (BD Pharmingen), Fascin Clone 55K2 (Millipore), S100A8/A9 or Calgranulin clone MAC387 mouse monoclonal (Dako) for human tissues, S100A9 (M-19) goat polyclonal antibody (Santacruz) for mouse tissues, anti-mouse and anti-human TNF-□ rabbit polyclonal antibodies (Rockland), rabbit polyclonal LTBP1 Atlas Ab2 (Sigma), Goat polyclonal anti-human CXCL1, C-15 (Santacruz). For senescence-associated β-galactosidase staining, unfixed cryosections were stained following manufacturer's protocol (Cell Signaling).

**Immunoblotting and Phospho-protein array profling.** Cell pellets were lysed with RIPA buffer and protein concentrations determined by BSA Protein Assay Kit (Biorad). Proteins were subsequently separated by SDS-PAGE and transferred to nitrocellulose membranes. Membranes were immunoblotted with antibodies against goat polyclonal antibodies from Santacruz namely S100A8 (M-19), S100A9 (M-19) used at 1:500, mouse monoclonal from Sigma against α-tubulin (clone B-5-1-2) used at 1:5000, rabbit polyclonal phospho-p65 (Ser 536), Phospho-Akt (Ser473) Clone D9E, rabbit polyclonal Phospho-Erk1/2 (Thr202/Tyr 204), rabbit polyclonal phospho-p38 (Thr180/Tyr182), rabbit Phospho-p70S6K (Thr389) Clone 108D2, rabbit polyclonal Phospho-p70S6K (Thr421/Ser424), rabbit Phospho-p70S6 (Ser235/236) Clone D57.2.2E, rabbit total p70S6 kinase Clone 49D7 all from Cell Signaling and used at 1:1000, rabbit polyclonal from Santacruz against IκBα (C-21) used at 1:500. For analysis of phosphorylation profiles of kinases, LM2 metastatic breast cancer cells were treated with recombinant human S100A8/A9 for 3hrs. Cells were subsequently lysed in NP-40 lysis buffer (10mM Tris pH7.4, 150mM NaCl, 4mM EDTA, 1% NP-40, 1mM sodium vanadate, 10mM sodium fluoride, with protease inhibitors). Lysates were probed using the human
Phospho-Kinase array blot (R&D Systems; Catalog # ARY003) according to manufacturer's instructions. The full list of proteins is available upon request and also on the manufacturer's website.

**Animal studies.** All experiments using animals were done in accordance to a protocol approved by MSKCC Institutional Animal Care and Use Committee (IACUC). *S100a9*+/+ and *S100a9*-/-mice (Hobbs et al., 2003), NOD-SCID NCR (NCI), athymic NCR nu/nu (Harlan), NIHIII homozygous nu/nu (Charles River), FVB/N (Charles River) female mice aged between 5-7 weeks were used for animal experiments. Primary PyMT cells were isolated from 15-wk old MMTV driven-polyoma virus middle T transgenic mice (Kim et al., 2009). PyMT cells were subsequently implanted into syngeneic FVB/N mice. Orthotopic metastasis assay has been described before (Minn et al., 2005). Briefly, PyMT, MDA231-LM2 or CN34LM1 cells were injected bilaterally into the 4th mammary fat pad of anesthetized mice (ketamine 100mg/kg/xylazine 10mg/kg). 500,000 cells were injected in 50µL volume PBS/matrigel mix (1:1). Matrigel used was growth factor reduced (BD Biosciences). Mammary tumor growth was monitored and growth was measured weekly using a digital caliper. After 6 weeks, mice were sacrificed and metastasis determined in lungs by ex vivo imaging. Lung colonization assays were as described previously (Minn et al., 2005). Briefly, lung colonization assays were performed by injecting 200,000 MDA231-LM2 (suspended in 100 µL PBS) into the lateral tail vein. Lung colonization was studied and determined by in-vivo bioluminescence imaging (BLI). Anesthetized mice (ketamine/xylazine) were injected retro-orbitally with D-Luciferin (150mg/kg) and imaged with IVIS Spectrum Xenogen machine (Caliper Life Sciences). Bioluminescence analysis was performed using Living Image software, version 2.50. For experiments involving CXCR2 inhibitor, NOD/SCID or athymic mice were injected intraperitoneally with either PEG400 (vehicle) or with SB-265610 (CXCR2 antagonist) purchased from Tocris at a dose of 2mg/kg body weight for five days a week administered once daily. For experiments involving MMP inhibitor, NOD/SCID or athymic mice were injected intraperitoneally with either PEG400 (vehicle) or with BB2516 (Marimastat) purchased from Tocris at a dose of either 8.7 or 4.4 mg/kg body weight for five days a week administered daily. For all experiments involving chemotherapy treatment, mice were injected once a week with either PBS vehicle, a combination of doxorubicin hydrochloride (Sigma) and cyclophosphamide monohydrate (Sigma) at a dose of 2mg/kg body wt and 60mg/kg body wt, respectively or Paclitaxel (Hospira) at a dose of 20mg/kg body wt or Methotrexate (Bedford Labs) at 5mg/body wt or 5-Fluorouracil (APP Pharmaceuticals) at 30 mg/kg body wt for the duration indicated in the regimen. For experiments involving antibody against TNF-α□ (Infliximab/Remicade from Janssen Biotech, Inc.), mice bearing CN34LM1 tumors were treated once a week intraperitoneally starting at 10 weeks post tumor inoculation and continued for five weeks until endpoint with the following regimen. Treatments included either PBS vehicle, a combination of doxorubicin hydrochloride (Sigma) and cyclophosphamide monohydrate (Sigma) at a dose of 2mg/kg body wt and 60mg/kg body wt either with or without anti-TNF-a blocking antibody (Remicade) at a dose of 10mg/kg body wt.
**Bone marrow harvest and transplantation.** Bone marrow cells were harvested from donor *S100a9*+/+ and *S100a9*-/-mice (Hobbs et al., 2003) by flushing femurs with sterile PBS containing penicillin/streptomycin/fungizone. Cells were washed 2X with sterile HBSS, dissociated with 18g needles and filtered through 70µm nylon mesh. For transplantation experiments, 2X10⁶ of the freshly isolated bone marrow cells from male donor mice were injected via tail-vein into irradiated female recipient NIHIII (B, T and NK cell deficient) mice. Radiation dose used was a total of 9 Gy in two split doses. Sulfatrim antibiotics were added to food following the transplant procedure. Immune reconstitution was assessed from blood smears by X and Y FISH analysis (Gopalan et al., 2009). After successful engrafting, mice were injected with LM2 cancer cells in mammary fat pad assays as described in the previous sections.

**Patient samples.** Paraffin embedded tissue microarrays containing primary breast cancer samples (IMH-364) and lymph node metastases (BRM481) used for FISH analysis were purchased from Imgenex and Pantomics, respectively. Basic clinical information and H&E are available on the manufacturer's website. Paraffin embedded tissue microarrays from lung metastases, sections from lung metastases and primary breast tumor cores before and after chemotherapy treatment were acquired from the MSKCC Department of Pathology in compliance with protocols approved by the MSKCC Institutional Review Board (IRB). TMA slides were baked for 1h at 56°C and immunostained for S100A8/A9 and TNF-α expression following procedures described in the Histological Staining section. Total immunoreactivity of both stainings were evaluated and scored by a clinical pathologist (E.B) in a blinded fashion.

**Bioinformatic analysis.** All bioinformatic analyses were conducted in R. Microarray data from human tumor data sets were processed as described (Zhang et al., 2009). The microarray data from cell lines (GSE2603 (Minn et al., 2005)) were processed with GCRMA together with updated probe set definitions using R packages *affy, gcrma* and *hs133ahsentrezgcdf* (version 10). Correlation between *CXCL1* (probe set 204470_at) and other genes was measured as the mean of Pearson's correlation coefficients from 3 independent microarray data sets for primary breast cancer: MSK/EMC368 (GSE2603 (Minn et al., 2005)) and (GSE2034 (Wang et al., 2005)), EMC189 (GSE5327(Minn et al., 2007)), and EMC58 (GSE12276 ((Bos et al., 2009)) and for metastases: GSE14020) in a cohort of 67 metastatic breast cancer samples from different sites (Zhang et al., 2009). Genes with extracellular function were selected by filtering out genes that did not belong to the Gene Ontology category Extracellular Space (GO:0005615). All heatmaps were generated by the *heatmap.2* function in the R package *gplots.*

**Statistical analysis.** Survival curves for patients were calculated using Kaplan-Meier method and differences between the curves were determined by log rank test. Synergy between individual pharmacological agents was assessed by comparing the observed data from a combination-treatment group to a simulated additive effect that was calculated as a product of median effects of individual drugs used as single agents. Comparison of means within groups in lung metastasis assays was analyzed using two-tailed unpaired Student's T test. Differences in TNF-α and S100A8/A9 (Calgranulin) expression in staining in patient tumors before and after chemotherapy were analyzed using Wilcoxon paired test (two-tailed). All other experiments were analyzed using two-sided Wilcoxon rank-sum test or unpaired two-sided t-test without unequal variance assumption unless specified. P values ≤ 0.05 were considered significant.

Table 3 shows quantitation of immune cell infiltrates expressed as percentages of total CD45+ leukocytes using indicated surface markers in transplant tumors with PyMT-F tumor cells expressing either shRNA control or sh*CXCL1*/*2* harvested at 5 weeks post tumor inoculation. Percentages are shown from the same tumor and are representative of three independent experiments.

Table 4 shows quantitation of % of positive cells expressing the indicated surface markers within the gated CD45+CD11b+Ly6G+ granulocytic-MDSC population from a PyMT-F tumor. Data are representative of two independent experiments.

Table 5 lists genes that correlate with *CXCL1* with a correlation coefficient of >0.3 and have extracellular gene products in 615 primary breast cancers based on microarray gene expression datasets.

Table 6 lists genes that correlate with *CXCL1* with a correlation coefficient of >0.3 and have extracellular gene products in breast cancer metastases based on microarray gene expression datasets.

Table 7 summarizes clinical information of breast cancer patients treated with neoadjuvant chemotherapy. Abbreviations used: NED- No evidence of disease, AWD- Alive with disease, CR-Complete response, PR-Partial response, MR-Minimal response, AC- Doxorubicin-Cyclophosphamide chemotherapy, T- Paclitaxel, T*-Albumin bound Paclitaxel, E- Epirubicin, HTrastuzumab, B-Bevacizumab, D+C-Docetaxel + Cyclophosphamide, T+L-Paclitaxel+Lapatinib.

### REFERENCES

1. Amiri, K.I., and Richmond, A. (2003). Fine tuning the transcriptional regulation of the CXCL1 chemokine. Prog Nucleic Acid Res Mol Biol 74, 1-36.
2. Balkwill, F. (2004). Cancer and the chemokine network. Nat Rev Cancer 4, 540-550.
3. Bean, J., Riely, G.J., Balak, M., Marks, J.L., Ladanyi, M., Miller, V.A., and Pao, W. (2008). Acquired resistance to epidermal growth factor receptor kinase inhibitors associated with a novel T854A mutation in a patient with EGFR-mutant lung adenocarcinoma. Clin Cancer Res 14, 7519-7525.
4. Beroukhim, R., Mermel, C.H., Porter, D., Wei, G., Raychaudhuri, S., Donovan, J., Barretina, J., Boehm, J.S., Dobson, J., Urashima, M., et al. (2010). The landscape of somatic copy-number alteration across human cancers. Nature 463, 899-905.
5. Bieche, I., Chavey, C., Andrieu, C., Busson, M., Vacher, S., Le Corre, L., Guinebretiere, J.M., Burlinchon, S., Lidereau, R., and Lazennec, G. (2007). CXC chemokines located in the 4q21 region are up-regulated in breast cancer. Endocr Relat Cancer 14, 1039-1052.
6. Bierhaus, A., Humpert, P.M., Morcos, M., Wendt, T., Chavakis, T., Arnold, B., Stern, D.M., and Nawroth, P.P. (2005). Understanding RAGE, the receptor for advanced glycation end products. J Mol Med (Berl) 83, 876-886.
7. Bos, P.D., Zhang, X.H., Nadal, C., Shu, W., Gomis, R.R., Nguyen, D.X., Minn, A.J., van de Vijver, M.J., Gerald, W.L., Foekens, J.A., et al. (2009). Genes that mediate breast cancer metastasis to the brain. Nature 459, 1005-1009.
8. Busch-Petersen, J. (2006). Small molecule antagonists of the CXCR2 and CXCR1 chemokine receptors as therapeutic agents for the treatment of inflammatory diseases. Curr Top Med Chem 6, 1345-1352.
9. Cailleau, R., Young, R., Olive, M., and Reeves, W.J., Jr. (1974). Breast tumor cell lines from pleural effusions. J Natl Cancer Inst 53, 661-674.
10. Chapman, R.W., Phillips, J.E., Hipkin, R.W., Curran, A.K., Lundell, D., and Fine, J.S. (2009). CXCR2 antagonists for the treatment of pulmonary disease. Pharmacol Ther 121, 55-68.
11. Condeelis, J., and Pollard, J.W. (2006). Macrophages: obligate partners for tumor cell migration, invasion, and metastasis. Cell 124, 263-266.
12. Coppe, J.P., Patil, C.K., Rodier, F., Sun, Y., Munoz, D.P., Goldstein, J., Nelson, P.S., Desprez, P.Y., and Campisi, J. (2008). Senescence-associated secretory phenotypes reveal cell-nonautonomous functions of oncogenic RAS and the p53 tumor suppressor. PLoS Biol 6, 2853-2868.
13. DeNardo, D.G., Andreu, P., and Coussens, L.M. (2010). Interactions between lymphocytes and myeloid cells regulate pro- versus anti-tumor immunity. Cancer Metastasis Rev 29, 309-316.
14. DeNardo, D.G., Barreto, J.B., Andreu, P., Vasquez, L., Tawfik, D., Kolhatkar, N., and Coussens, L.M. (2009). CD4(+) T cells regulate pulmonary metastasis of mammary carcinomas by enhancing protumor properties of macrophages. Cancer Cell 16, 91-102.
15. DeNardo, D.G., Brennan DJ, Rexhepaj E, Ruffell B, Shiao SL, Madden SF, Gallagher, WM, Wadhwani N, Keil SD, Junaid SA, Rugo HS, Hwang ES, Jirstrom K, West BL, Coussens LM (2011). Leukocyte Complexity Predicts Breast Cancer Survival and Functionally Regulates Response to Chemotherapy In Cancer Discovery.
16. Ebos, J.M., Lee, C.R., Cruz-Munoz, W., Bjarnason, G.A., Christensen, J.G., and Kerbel, R.S. (2009). Accelerated metastasis after short-term treatment with a potent inhibitor of tumor angiogenesis. Cancer Cell 15, 232-239.
17. Gabrilovich, D.I., and Nagaraj, S. (2009). Myeloid-derived suppressor cells as regulators of the immune system. Nat Rev Immunol 9, 162-174.
18. Gebhardt, C., Nemeth, J., Angel, P., and Hess, J. (2006). S100A8 and S100A9 in inflammation and cancer. Biochem Pharmacol 72, 1622-1631.
19. Ghavami, S., Rashedi, I., Dattilo, B.M., Eshraghi, M., Chazin, W.J., Hashemi, M., Wesselborg, S., Kerkhoff, C., and Los, M. (2008). S100A8/A9 at low concentration promotes tumor cell growth via RAGE ligation and MAP kinase-dependent pathway. J Leukoc Biol 83, 1484-1492.
20. Gilbert, L.A., and Hemann, M.T. (2010). DNA damage-mediated induction of a chemoresistant niche. Cell 143, 355-366.
21. Gomis, R.R., Alarcon, C., Nadal, C., Van Poznak, C., and Massague, J. (2006). C/EBPbeta at the core of the TGFbeta cytostatic response and its evasion in metastatic breast cancer cells. Cancer Cell 10, 203-214.
22. Gonzalez-Angulo, A.M., Morales-Vasquez, F., and Hortobagyi, G.N. (2007). Overview of resistance to systemic therapy in patients with breast cancer. Adv Exp Med Biol 608, 1-22.
23. Gopalan, A., Leversha, M.A., Satagopan, J.M., Zhou, Q., Al-Ahmadie, H.A., Fine, S.W., Eastham, J.A., Scardino, P.T., Scher, H.I., Tickoo, S.K., et al. (2009). TMPRSS2-ERG gene fusion is not associated with outcome in patients treated by prostatectomy. Cancer Res 69, 1400-1406.
24. Grivennikov, S., Karin, E., Terzic, J., Mucida, D., Yu, G.Y., Vallabhapurapu, S., Scheller, J., Rose-John, S., Cheroutre, H., Eckmann, L., et al. (2009). IL-6 and Stat3 are required for survival of intestinal epithelial cells and development of colitis-associated cancer. Cancer Cell 15, 103-113.
25. Grivennikov, S.I., Greten, F.R., and Karin, M. (2010). Immunity, inflammation, and cancer. Cell 140, 883-899.
26. Gupta, G.P., Nguyen, D.X., Chiang, A.C., Bos, P.D., Kim, J.Y., Nadal, C., Gomis, R.R., Manova-Todorova, K., and Massague, J. (2007). Mediators of vascular remodelling coopted for sequential steps in lung metastasis. Nature 446, 765-770.
27. Hanahan, D., and Weinberg, R.A. (2011). Hallmarks of cancer: the next generation. Cell 144, 646-674.
28. Hermani, A., De Servi, B., Medunjanin, S., Tessier, P.A., and Mayer, D. (2006). S100A8 and S100A9 activate MAP kinase and NF-kappaB signaling pathways and trigger translocation of RAGE in human prostate cancer cells. Exp Cell Res 312, 184-197.
29. Hiratsuka, S., Watanabe, A., Aburatani, H., and Maru, Y. (2006). Tumour-mediated upregulation of chemoattractants and recruitment of myeloid cells predetermines lung metastasis. Nat Cell Biol 8, 1369-1375.
30. Hiratsuka, S., Watanabe, A., Sakurai, Y., Akashi-Takamura, S., Ishibashi, S., Miyake, K., Shibuya, M., Akira, S., Aburatani, H., and Maru, Y. (2008). The S100A8-serum amyloid A3-TLR4 paracrine cascade establishes a pre-metastatic phase. Nat Cell Biol 10, 1349- 1355.
31. Hobbs, J.A., May, R., Tanousis, K., McNeill, E., Mathies, M., Gebhardt, C., Henderson, R., Robinson, M.J., and Hogg, N. (2003). Myeloid cell function in MRP-14 (S100A9) null mice. Mol Cell Biol 23, 2564-2576.
32. Horuk, R. (2009). Chemokine receptor antagonists: overcoming developmental hurdles. Nat Rev Drug Discov 8, 23-33.
33. Hsieh, H.L., Schafer, B.W., Sasaki, N., and Heizmann, C.W. (2003). Expression analysis of S100 proteins and RAGE in human tumors using tissue microarrays. Biochem Biophys Res Commun 307, 375-381.
34. Hu, G., Chong, R.A., Yang, Q., Wei, Y., Blanco, M.A., Li, F., Reiss, M., Au, J.L., Haffty, B.G., and Kang, Y. (2009). MTDH activation by 8q22 genomic gain promotes chemoresistance and metastasis of poor-prognosis breast cancer. Cancer Cell 15, 9-20.
35. Ichikawa, M., Williams, R., Wang, L., Vogl, T., and Srikrishna, G. (2011). S100A8/A9 activate key genes and pathways in colon tumor progression. Mol Cancer Res 9, 133-148.
36. Ijichi, H., Chytil, A., Gorska, A.E., Aakre, M.E., Bierie, B., Tada, M., Mohri, D., Miyabayashi, K., Asaoka, Y., Maeda, S., et al. (2011). Inhibiting Cxcr2 disrupts tumorstromal interactions and improves survival in a mouse model of pancreatic ductal adenocarcinoma. J Clin Invest 121, 4106-4117.
37. Jones, S.E. (2008). Metastatic breast cancer: the treatment challenge. Clin Breast Cancer 8, 224-233.
38. Joyce, J.A., and Pollard, J.W. (2009). Microenvironmental regulation of metastasis. Nat Rev Cancer 9, 239-252.
39. Karnoub, A.E., Dash, A.B., Vo, A.P., Sullivan, A., Brooks, M.W., Bell, G.W., Richardson, A.L., Polyak, K., Tubo, R., and Weinberg, R.A. (2007). Mesenchymal stem cells within tumour stroma promote breast cancer metastasis. Nature 449, 557-563.
40. Kim, M.Y., Oskarsson, T., Acharyya, S., Nguyen, D.X., Zhang, X.H., Norton, L., and Massague, J. (2009). Tumor self-seeding by circulating cancer cells. Cell 139, 1315-1326.
41. Leversha, M.A. (2001). Mapping of genomic clones by fluorescence in situ hybridization. Methods Mol Biol 175, 109-127.
42. Lin, E.Y., Jones, J.G., Li, P., Zhu, L., Whitney, K.D., Muller, W.J., and Pollard, J.W. (2003). Progression to malignancy in the polyoma middle T oncoprotein mouse breast cancer model provides a reliable model for human diseases. Am J Pathol 163, 2113-2126.
43. Minn, A.J., Gupta, G.P., Padua, D., Bos, P., Nguyen, D.X., Nuyten, D., Kreike, B., Zhang, Y., Wang, Y., Ishwaran, H., et al. (2007). Lung metastasis genes couple breast tumor size and metastatic spread. Proc Natl Acad Sci U S A 104, 6740-6745.
44. Minn, A.J., Gupta, G.P., Siegel, P.M., Bos, P.D., Shu, W., Giri, D.D., Viale, A., Olshen, A.B., Gerald, W.L., and Massague, J. (2005). Genes that mediate breast cancer metastasis to lung. Nature 436, 518-524.
45. Montero, A.J., Diaz-Montero, C.M., Deutsch, Y.E., Hurley, J., Koniaris, L.G., Rumboldt, T., Yasir, S., Jorda, M., Garret-Mayer, E., Avisar, E., et al. (2011). Phase 2 study of neoadjuvant treatment with NOV-002 in combination with doxorubicin and cyclophosphamide followed by docetaxel in patients with HER-2 negative clinical stage II-IIIc breast cancer. Breast Cancer Res Treat.
46. Morris, P.G., McArthur, H.L., and Hudis, C.A. (2009). Therapeutic options for metastatic breast cancer. Expert Opin Pharmacother 10, 967-981.
47. Muller, A., Homey, B., Soto, H., Ge, N., Catron, D., Buchanan, M.E., McClanahan, T., Murphy, E., Yuan, W., Wagner, S.N., et al. (2001). Involvement of chemokine receptors in breast cancer metastasis. Nature 410, 50-56.
48. Murdoch, C., Muthana, M., Coffelt, S.B., and Lewis, C.E. (2008). The role of myeloid cells in the promotion of tumour angiogenesis. Nat Rev Cancer 8, 618-631.
49. Nam, J.S., Kang, M.J., Suchar, A.M., Shimamura, T., Kohn, E.A., Michalowska, A.M., Jordan, V.C., Hirohashi, S., and Wakefield, L.M. (2006). Chemokine (C-C motif) ligand 2 mediates the prometastatic effect of dysadherin in human breast cancer cells. Cancer Res 66, 7176-7184.
50. Ostrand-Rosenberg, S. (2010). Myeloid-derived suppressor cells: more mechanisms for inhibiting antitumor immunity. Cancer Immunol Immunother 59, 1593-1600.
51. Ostrand-Rosenberg, S., and Sinha, P. (2009). Myeloid-derived suppressor cells: linking inflammation and cancer. J Immunol 182, 4499-4506.
52. Paez-Ribes, M., Allen, E., Hudock, J., Takeda, T., Okuyama, H., Vinals, F., Inoue, M., Bergers, G., Hanahan, D., and Casanovas, O. (2009). Antiangiogenic therapy elicits malignant progression of tumors to increased local invasion and distant metastasis. Cancer Cell 15, 220-231.
53. Pegram, M.D., Konecny, G.E., O'Callaghan, C., Beryt, M., Pietras, R., and Slamon, D.J. (2004). Rational combinations of trastuzumab with chemotherapeutic drugs used in the treatment of breast cancer. J Natl Cancer Inst 96, 739-749.
54. Poulikakos, P.I., Persaud, Y., Janakiraman, M., Kong, X., Ng, C., Moriceau, G., Shi, H., Atefi, M., Titz, B., Gabay, M.T., et al. (2011). RAF inhibitor resistance is mediated by dimerization of aberrantly spliced BRAF(V600E). Nature.
55. Qian, B.Z., Li, J., Zhang, H., Kitamura, T., Zhang, J., Campion, L.R., Kaiser, E.A., Snyder, L.A., and Pollard, J.W. (2011). CCL2 recruits inflammatory monocytes to facilitate breast-tumour metastasis. Nature 475, 222-225.
56. Raman, D., Baugher, P.J., Thu, Y.M., and Richmond, A. (2007). Role of chemokines in tumor growth. Cancer Lett 256, 137-165.
57. Roodhart, J.M., Daenen, L.G., Stigter, E.C., Prins, H.J., Gerrits, J., Houthuijzen, J.M., Gerritsen, M.G., Schipper, H.S., Backer, M.J., van Amersfoort, M., et al. (2011). Mesenchymal stem cells induce resistance to chemotherapy through the release of platinum-induced fatty acids. Cancer Cell 20, 370-383.
58. Shah, N.P., Nicoll, J.M., Nagar, B., Gorre, M.E., Paquette, R.L., Kuriyan, J., and Sawyers, C.L. (2002). Multiple BCR-ABL kinase domain mutations confer polyclonal resistance to the tyrosine kinase inhibitor imatinib (STI571) in chronic phase and blast crisis chronic myeloid leukemia. Cancer Cell 2,,117-125.
59. Shaked, Y., Henke, E., Roodhart, J.M., Mancuso, P., Langenberg, M.H., Colleoni, M., Daenen, L.G., Man, S., Xu, P., Emmenegger, U., et al. (2008). Rapid chemotherapy induced acute endothelial progenitor cell mobilization: implications for antiangiogenic drugs as chemosensitizing agents. Cancer Cell 14, 263-273.
60. Shojaei, F., Wu, X., Malik, A.K., Zhong, C., Baldwin, M.E., Schanz, S., Fuh, G., Gerber, H.P., and Ferrara, N. (2007). Tumor refractoriness to anti-VEGF treatment is mediated by CD11b+Gr1+ myeloid cells. Nat Biotechnol 25, 911-920.
61. Shree, T., Olson, O.C., Elie, B.T., Kester, J.C., Garfall, A.L., Simpson, K., Bell-McGuinn, K.M., Zabor, E.C., Brogi, E., and Joyce, J.A. (2011). Macrophages and cathepsin proteases blunt chemotherapeutic response in breast cancer. Genes Dev 25, 2465-2479.
62. Sinha, P., Okoro, C., Foell, D., Freeze, H.H., Ostrand-Rosenberg, S., and Srikrishna, G. (2008). Proinflammatory S100 proteins regulate the accumulation of myeloid-derived suppressor cells. J Immunol 181, 4666-4675.
63. Steward, W.P., and Thomas, A.L. (2000). Marimastat: the clinical development of a matrix metalloproteinase inhibitor. Expert Opin Investig Drugs 9, 2913-2922.
64. Stewart, T.J., and Abrams, S.I. (2007). Altered immune function during long-term host tumor interactions can be modulated to retard autochthonous neoplastic growth. J Immunol 179, 2851-2859.
65. Stillie, R., Farooq, S.M., Gordon, J.R., and Stadnyk, A.W. (2009). The functional significance behind expressing two IL-8 receptor types on PMN. J Leukoc Biol 86, 529- 543.
66. Tan, W., Zhang, W., Strasner, A., Grivennikov, S., Cheng, J.Q., Hoffman, R.M., and Karin, M. (2011). Tumour-infiltrating regulatory T cells stimulate mammary cancer metastasis through RANKL-RANK signalling. Nature 470, 548-553.

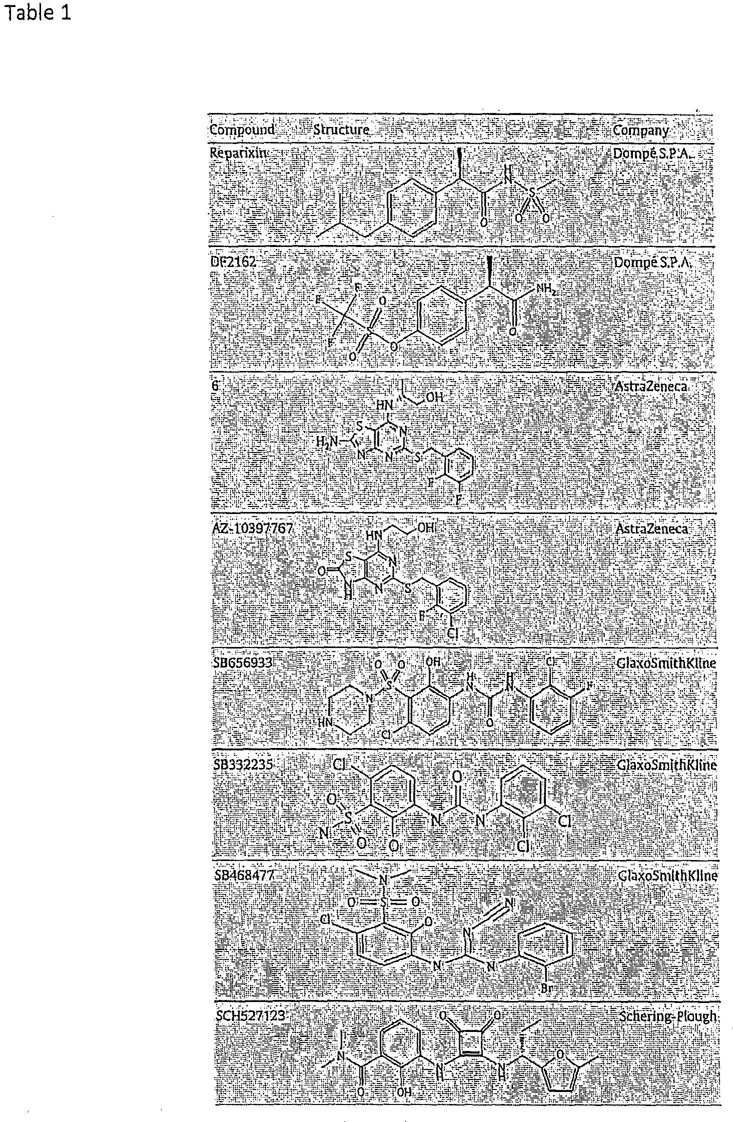

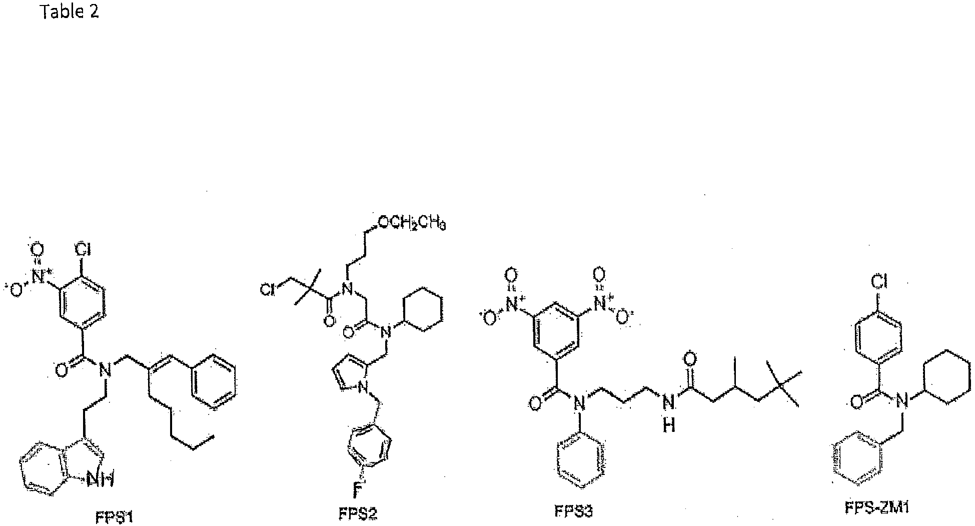

**Table 3**

| **Surface markers** | **sh RNA control** | **sh Cxcl1/2** |
|---|---|---|
| F4/80⁺ | 31.8 | 35.6 |
| CD4⁺ | 1.08 | 0.651 |
| CD8⁺ | 0.157 | 0.062 |
| CD3⁺CD25⁺ | 0.0536 | 0.0481 |
| CD3⁺gdTCR⁺ | 0.268 | 0.353 |
| CD3⁻CD49⁺ | 0.926 | 2.08 |
| B220⁺ | 4.88 | 7.09 |

**Table 4**

| **Surface markers** | **% positive cells** |
|---|---|
| CD80 | 5.8 |
| CD86 | 2.11 |
| F4/80 | 12.3 |
| CD117 | 2.94 |
| IL4Rα | 1.22 |
| VEGFR1 | 0.175 |
| CD34 | 0.309 |
| Sca1 | 24.7 |

**Table 5**

| **No.** | **Probe** | **Gene symbol** | **Gene title** | **MeanCXCL1 Correlation** | **Protein type** |
|---|---|---|---|---|---|
| 1 | 204470_at | CXCL1 | chemokine (C-X-C motif) ligand 1 (melanoma growth stimulating activity, alpha) | 1 | Chemokine |
| 2 | 209774_x_at | CXCL2 | chemokine (C-X-C motif) ligand 2 | 0.58420139 | Chemokine |
| 3 | 205476 at | CCL20 | chemokine (C-C motif) ligand 20 | 0.507142691 | Chemokine |
| 4 | 214974_x_at | CXCL5 | chemokine (C-X-C motif) ligand 5 | 0.503264274 | Chemokine |
| 5 | 207850_at | CXCL3 | chemokine (C-X-C motif) ligand 3 | 0.442087948 | Chemokine |
| 6 | 202917_s_at | S100A8 | calcium binding protein A8 | 0.436591522 | Chemokine |
| 7 | 219454_at | EGFL6 | EGF-like-domain, multiple 6 | 0.430470932 | Growth factor |
| 8 | 203535_at | S100A9 | S100 calcium binding protein A9 | 0.421452025 | Chemokine |
| 9 | 214370_at | S100A8 | S100 calcium binding protein A8 | 0.416780713 | Chemokine |
| 10 | 201984_s_at | EGFR | epidermal growth factor receptor (erythroblastic leukemia viral (verb-b) oncogene homolog, avian) | 0.414932963 | Receptor |
| 11 | 216598 s at | CCL2 | chemokine (C-C motif) ligand 2 | 0.406927087 | Chemokine |
| 12 | 32128_at | CCL18 | chemokine (C-C motif) ligand 18 (pulmonary and activation-regulated) | 0.404640555 | Chemokine |
| 13 | 206336_at | CXCL6 | chemokine (C-X-C motif) ligand 6 (granulocyte chemotactic protein 2) | 0.398592045 | Chemokine |
| 14 | 209924_at | CCL18 | chemokine (C-C motif) ligand 18 (pulmonary and activation-regulated) | 0.397682961 | Chemokine |
| 15 | 204259_at | MMP7 | matrix metallopeptidase 7 (matrilysin, uterine) | 0.392437116 | Enzyme |
| 16 | 204475_at | MMP1 | matrix metallopeptidase 1 (interstitial collagenase) | 0.387440495 | Enzyme |
| 17 | 211506_s_at | IL8 | interleukin 8 | 0.379937059 | Chemokine |
| 18 | 201983_s_at | EGFR | epidermal growth factor receptor (erythroblastic leukemia viral (verb-b) oncogene homolog, avian) | 0.376102229 | Growth factor receptor |
| 19 | 39402_at | IL1B | interleukin 1, beta | 0.3687253 | Cytokine |
| 20 | 205207_at | IL6 | interleukin 6 (interferon, beta 2) | 0.367706402 | Cytokine |
| 21 | 209395_at | CHI3L1 | chitinase 3-like 1 (cartilage glycoprotein-39) | 0.363640348 | Glycoprotein |
| 22 | 213060_s_at | CHI3L2 | chitinase 3-like 2 | 0.361544619 | Glycoprotein |
| 23 | 204304_s_at | PROM1 | prominin 1 | 0.356858262 | Glycoprotein |
| 24 | 206407_s_at | CCL13 | chemokine (C-C motif) ligand 13 | 0.352624539 | Chemokine |
| 25 | 204655 at | CCL5 | chemokine (C-C motif) ligand 5 | 0.349987181 | Chemokine |
| 26 | 209396_s_at | CHI3L1 | chitinase 3-like 1 (cartilage glycoprotein-39) | 0.348925927 | Glycoprotein |
| 27 | 205992_s_at | IL 15 | interleukin 15 | 0.343998585 | Cytokine |
| 28 | 204533_at | CXCL10 | chemokine (C-X-C motif) ligand 10 | 0.342768882 | Cytokine |
| 29 | 218995_s_at | EDN1 | endothelin 1 | 0.336944246 | Peptide |
| 30 | 201859_at | SRGN | serglycin | 0.336477656 | Proteoglycan |
| 31 | 205067_at | IL1B | interleukin 1, beta | 0.332933317 | cytokine |
| 32 | 203828_s_at | IL32 | interleukin 32 | 0.331005112 | Cytokine |
| 33 | 1405_i_at | CCL5 | chemokine (C-C motif) ligand 5 | 0.330034012 | Chemokine |
| 34 | 202912_at | ADM | adrenomedullin | 0.322009119 | Preprohormone |
| 35 | 213975_s_at | LYZ | (renal amyloidosis) | 0.32161241 | Enyzme |
| 36 | 207113_s_at | TNF | tumor necrosis factor (TNF superfamily, member 2) | 0.318836447 | Cytokine |
| 37 | 207339_s_at | LTB | beta (TNF superfamily, member 3) | 0.313814703 | Cytokine |
| 38 | 823_at | CX3CL1 | chemokine (C-X3-C motif) ligand 1 | 0.311766873 | Chemokine |
| 39 | 205290_s_at | BMP2 | bone morphogenetic protein 2 | 0.309658146 | Cytokine |
| 40 | 214456_x_at | SAA1 /// SAA2 | serum amyloid A1 /// serum amyloid A2 | 0.307759706 | Apolipoprotein |
| 41 | 202510_s_at | TNFAIP2 | tumor necrosis factor, alpha-induced protein 2 | 0.307469211 | TNF induced primary response gene |
| 42 | 208607_s_at | SAA1 /// SAA2 | serum amyloid A1 /// serum amyloid A2 | 0.306284948 | Apolipoprotein |
| 43 | 201858_s_at | SRGN | serglycin | 0.304778725 | Proteoglycan |
| 44 | 214038_at | CCL8 | chemokine (C-C motif) ligand 8 | 0.301369728 | Chemokine |

**Table 6**

| **No.** | **Probe** | **Gene symbol** | **Gene title** | **MeanCXCL1 correlation** | **Protein type** |
|---|---|---|---|---|---|
| 1 | 204470_at | CXCL1 | chemokine (C-X-C motif) ligand 1 (melanoma growth stimulating activity, alpha) | 1 | Chemokine |
| 2 | 207850_at | CXCL3 | chemokine (C-X-C motif) ligand 3 | 0.727384488 | Chemokine |
| 3 | 214974_x_at | CXCL5 | chemokine (C-X-C motif) ligand 5 | 0.72156911 | Chemokine |
| 4 | 214456_x_at | SAA1 /// SAA2 | serum amyloid A1 /// serum amyloid A2 | 0.692151247 | Apolipoprotein |
| 5 | 208607_s_at | sAA1 /// SAA2 | serum amyloid A1 /// serum amyloid A2 | 0.62306514 | Apolipoprotein |
| 6 | 202859_x_at | IL8 | interleukin 8 | 0.618285539 | Chemokine |
| 7 | 204304_s_at | PROM1 | prominin 1 | 0.574115805 | Glycoprotein |
| 8 | 206336_at | CXCL6 | chemokine (C-X-C motif) ligand 6 (granulocyte chemotactic protein 2) | 0.518174074 | Chemokine |
| 9 | 215101_s_at | CXCL5 | chemokine (C-X-C motif) ligand 5 | 0.501422376 | Chemokine |
| 10 | 204259_at | MMP7 | matrix metallopeptidase 7 (matrilysin, uterine) | 0.479886003 | Enzyme |
| 11 | 209774_x_at | CXCL2 | chemokine (C-X-C motif) ligand 2 | 0.46608999 | Chemokine |
| 12 | 203535_at | S100A9 | S100 calcium binding protein A9 | 0.460754596 | Chemokine |
| 13 | 211506_s_at | IL8 | interleukin 8 | 0.446340158 | Chemokine |
| 14 | 203687_at | CX3CL1 | chemokine (C-X3-C motif) ligand 1 | 0.440105848 | Chemokine |
| 15 | 823_at | CX3CL1 | chemokine (C-X3-C motif) ligand 1 | 0.439724863 | Chemokine |
| 16 | 209924_at | CCL18 | chemokine (C-C motif) ligand 18 (pulmonary and activation-regulated) | 0.437635648 | Chemokine |
| 17 | 33322_l_at | SFN | stratifin | 0.436301249 | DNA damage response protein |
| 18 | 206407_s_at | CCL13 | chemokine (C-C motif) ligand 13 | 0.433070003 | Chemokine |
| 19 | 202917_s_at | S100A8 | S100 calcium binding protein A8 | 0.432105649 | Chemokine |
| 20 | 209260_at | SFN | stratifin | 0.423454846 | DNA damage response protein |
| 21 | 205014_at | FGFBP1 | fibroblast growth factor binding protein 1 | 0.411514624 | Glycoprotein |
| 22 | 204455_at | DST | dystonin | 0.407415007 | Cytoskeletal linker protein |
| 23 | 213936_x_at | SFTPB | surfactant, pulmonary-associated protein B | 0.406917077 | Surfactant associated protein |
| 24 | 33323_r_at | SFN | stratifin | 0.406474275 | DNA damage response protein |
| 25 | 205476_at | CCL20 | chemokine (C-C motif) ligand 20 | 0.396314999 | Chemokine |
| 26 | 214354_x_at | SFTPB | surfactant, pulmonary-associated protein B | 0.393006377 | Surfactant associated protein |
| 27 | 32128_at | CCL18 | chemokine (C-C motif) ligand 18 (pulmonary and activation-regulated) | 0.392319189 | Chemokine |
| 28 | 1405_i_at | CCL5 | chemokine (C-C motif) ligand 5 | 0.389002998 | Chemokine |
| 29 | 209396_s_at | CHI3L 1 | chitinase 3-like 1 (cartilage glycoprotein-39) | 0.381922747 | Glycoprotein |
| 30 | 218995_s_at | EDN1 | endothelin 1 | 0.380452512 | Vasocontrictor peptide |
| | | | | | |
| 31 | 205266_at | LIF | leukemia inhibitory factor (cholinergic differentiation factor) | 0.379145451 | Cytokine |
| 32 | 201983_s_at | EGFR | epidermal growth factor receptor | 0.377765594 | Receptor |
| 33 | 201984_s_at | EGFR | epidermal growth factor receptor | 0.37549647 | Receptor |
| 34 | 209395_at | CHI3L1 | chitinase 3-like 1 (cartilage glycoprotein-39) | 0.367313688 | Glycoprotein |
| 35 | 202018_s_at | LTF | lactotransferrin | 0.363344816 | Glycoprotein |
| 36 | 209810_at | SFTPB | surfactant, pulmonary-associated protein B | 0.362288432 | Surfactant associated protein |
| 37 | 204153_s_at | MFNG | MFNG O-fucosylpeptide 3-beta-N-acetylglucosaminyltransferase | 0.360062212 | Glycoprotein |
| 38 | 204655_at | CCL5 | chemokine (C-C motif) ligand 5 | 0.355893728 | Chemokine |
| 39 | 214461_at | LBP | lipopolysaccharide binding protein | 0.350076877 | Acute phase protein |
| 40 | 206560_s_at | MIA | melanoma inhibitory activity | 0.349846506 | Growth factor |
| 41 | 37004_at | SFTPB | surfactant, pulmonary-associated protein B | 0.349391767 | Surfactant associated protein |
| 42 | 203828_s_at | IL32 | interleukin 32 | 0.345291084 | Cytokine |
| 43 | 212662_at | PVR | poliovirus receptor | 0.339601184 | Ig like family of proteins |
| 44 | 205654_at | C4BPA | complement component 4 binding protein, alpha | 0.335596848 | Complement activation protein |
| 45 | 207861_at | CCL22 | chemokine (C-C motif) ligand 22 | 0.332404796 | Chemokine |
| 46 | 207816_at | LALBA | lactalbumin, alpha- | 0.330824407 | Protein in lactose synthesis |
| 47 | 205016_at | TGFA | transforming growth factor, alpha | 0.31799294 | Growth factor |
| 48 | 214370_at | S100A8 | S100 calcium binding protein A8 | 0.313752555 | Chemokine |
| 49 | 219454_at | EGFL6 | EGF-like-domain, multiple 6 | 0.310355128 | Growth factor |
| 50 | 204858_s_at | TYMP | thymidine phosphorylase | 0.30100493 | Enzyme |

**Table 7**

| **Case No.** | **ER Status** | **PR Status** | **HER2 Status** | **Grade** | **Chemo** | **Pathologic al Response to Chemo** | **Surviv al Status** |
|---|---|---|---|---|---|---|---|
| 1001 | Positive | Positive | Negative | 3 | AC-T | PR | NED |
| 1002 | Negative | Negative | Negative | 3 | EC-T | PR | AWD |
| 1003 | Positive | Positive | Negative | 3 | AC-T | MR | NED |
| 1004 | Negative | Negative | Negative | 3 | AC-T | CR | NED |
| 1005 | Positive | Positive | Negative | 3 | AC-T | MR | AWD |
| 1006 | Negative | Negative | Negative | 3 | AC-T | PR | NED |
| 1007 | Negative | Negative | Positive | 3 | AC-TH | CR | NED |
| 1008 | Positive | Positive | Negative | 3 | AC-T | MR | NED |
| 1009 | Positive | Positive | Negative | 2 | AC-T | MR | NED |
| 1010 | Negative | Negative | Negative | 2 | AC-T | PR | NED |
| 1011 | Positive | Positive | Negative | 3 | AC-T | PR | NED |
| 1012 | Positive | Positive | Negative | 2 | AC-T | PR | NED |
| 1013 | Positive | Positive | Negative | 3 | AC-T | MR | NED |
| 1014 | Negative | Negative | Negative | 3 | AC-T | PR | NED |
| 1015 | Positive | Negative | Positive | 3 | AC-TH | MR | NED |
| 1016 | Positive | Positive | Negative | 1 | AC-T | MR | NED |
| 1017 | Positive | Negative | Negative | 3 | AC-T | PR | Died - other |
| 1018 | Positive | Positive | Negative | 2 | AC-Nab-paclitax el | PR | NED |
| 1019 | Negative | Negative | Positive | 3 | TH | CR | NED |
| 1020 | Negative | Negative | Positive | 3 | AC-TH | CR | NED |
| 1021 | Negative | Negative | Positive | 3 | AC-T | PR | Died - of disease |
| 1022 | Positive | Positive | Negative | 3 | AC-T | MR | NED |
| 1023 | Positive | Negative | Positive | 3 | AC-T | MR | NED |
| 1024 | Positive | Positive | Negative | 3 | AC-T | PR | NED |
| 1025 | Negative | Neg | Positive | 3 | EC-T | PR | AWD |
| 1026 | Positive | Negative | Negative | 3 | AC-T | CR | NED |
| 1027 | Positive | Positive | Positive | 3 | AC-TH | PR | NED |
| 1028 | Positive | Positive | Negative | 3 | AC-T | PR | NED |
| 1029 | Positive | Positive | Negative | 3 | AC-T | PR | NED |
| 1031 | Negative | Negative | Negative | 3 | Carbopl atin, T,B,AC+ B | PR | NED |
| 1032 | Positive | Positive | Positive | 3 | AC-TH | CR | NED |
| 1033 | Positive | Positive | Negative | 3 | AC-T | PR | NED |
| 1034 | Positive | Positive | Negative | 2 | EC-T | PR | NED |
| 1035 | Positive | Negative | Negative | 3 | AC-T | PR | NED |
| 1036 | Positive | Positive | Negative | Unkno wn | EC-T | PR | NED |
| 1037 | Positive | Positive | Negative | 3 | D+C | PR | NED |
| 1038 | Positive | Negative | Negative | 3 | T+L | PR | NED |
| 1039 | Negative | Negative | Negative | 3 | AC-T | CR | NED |
| 1040 | Positive | Positive | Positive | 3 | AC-TH | CR | NED |
| 1041 | Positive | Positive | Negative | 3 | AC-T | CR | NED |

## Claims

1. A method of selecting patients having lung or breast cancer that are suitable for a defined treatment, the method comprising the steps of:
(a) measuring the amount of S100A8/A9 protein present in samples previously obtained from patients having lung or breast cancer;
(b) assessing whether the measured amount of S100A8/A9 protein is less than a predetermined standard value, or above said predetermined standard value; and
(c) selecting patients from whose samples the measured amount is less than the predetermined standard value for administration of a cytotoxic chemotherapy agent, and selecting patients from whose samples the measured amount is above the predetermined standard value for administration of chemotherapy agents that are directed to one or more components of a TNF alpha - CXCL1/2 - S100A8/A9 survival axis; wherein said chemotherapy agents are directed to one or more targets selected from the group consisting of the chemokines CXCL1/2/3/8 and their receptors CXCR1/2, the cytokine TNF-alpha and its receptor TNFR, the factors S100A8/A9 and their receptors RAGE and TLR4.

2. The method of claim 1, wherein the samples are from tumour tissue.

3. The method of claim 1, wherein the samples are from serum.

4. A method according to any of claims 1 to 3, wherein the samples have previously been obtained from human patients suffering from lung or breast cancer to whom a cytotoxic chemotherapeutic agent has been previously administered.

5. A kit consisting of reagents for assessing responsiveness to chemotherapy treatments in a human breast cancer patient, said kit including:
(a) reagents for measurement of the amount of CXCL1/2 and/or the amount of TNF-alpha in a sample from the patient; and
(b) reagents for measurement of the amount of S100A8/A9 in a sample from the patient,
wherein the reagents are antibodies and/or oligonucleotide probes specific for CXCL1/2, TNF-alpha and/or S100A8/A9.

6. The kit of claim 5, which contains reagents for measuring the amount of CXCL1 and of CXCL2 in the sample.

## Patentansprüche

1. Ein Verfahren zum Auswählen von Patienten mit Lungen- oder Brustkrebs, die für eine definierte Behandlung geeignet sind, wobei das Verfahren die folgenden Schritte beinhaltet:
(a) Messen der Menge an S100A8/A9-Protein, die in Proben vorhanden ist, die zuvor von Patienten mit Lungen- oder Brustkrebs erhalten wurden;
(b) Beurteilen, ob die gemessene Menge an S100A8/A9-Protein geringer als ein vorbestimmter Standardwert oder höher als der vorbestimmte Standardwert ist; und
(c) Auswählen der Patienten, deren Proben eine gemessene Menge aufweisen, die geringer als der vorbestimmte Standardwert ist, für die Verabreichung eines zytotoxischen Chemotherapeutikums, und Auswählen der Patienten, deren Proben eine gemessene Menge aufweisen, die höher als der vorbestimmte Standardwert ist, für die Verabreichung von Chemotherapeutika, die auf eine oder mehrere Komponenten einer TNF-alpha-CXCL1/2-S100A8/A9-Überlebensachse ausgerichtet sind; wobei die Chemotherapika auf eines oder mehrere Ziele ausgerichtet sind, ausgewählt aus der Gruppe, bestehend aus den Chemokinen CXCL1/2/3/8 und ihren Rezeptoren CXCR1/2, dem Zytokin TNF-alpha und seinem Rezeptor TNFR, den Faktoren S100A8/A9 und ihren Rezeptoren RAGE und TLR4.

2. Verfahren gemäß Anspruch 1, wobei die Proben aus Tumorgewebe stammen.

3. Verfahren gemäß Anspruch 1, wobei die Proben aus Serum stammen.

4. Verfahren gemäß einem der Ansprüche 1 bis 3, wobei die Proben zuvor von menschlichen Patienten erhalten wurden, die an Lungen- oder Brustkrebs leiden, denen zuvor ein zytotoxisches Chemotherapeutikum verabreicht worden ist.

5. Ein Kit, bestehend aus Reagenzien zur Beurteilung des Ansprechens auf Chemotherapiebehandlungen in einem menschlichen Brustkrebspatienten, wobei der Kit Folgendes umfasst:
(a) Reagenzien zur Messung der Menge an CXCL1/2 und/oder der Menge an TNF-alpha in einer Probe von dem Patienten; und
(b) Reagenzien zur Messung der Menge an S100A8/A9 in einer Probe von dem Patienten, wobei die Reagenzien Antikörper- und/oder Oligonukleotidsonden sind, die für CXCL1/2, TNF-alpha und/oder S100A8/A9 spezifisch sind.

6. Kit gemäß Anspruch 5, der Reagenzien zur Messung der Menge an CXCL1 und von CXCL2 in der Probe enthält.

## Revendications

1. Méthode de sélection de patients atteints d'un cancer du poumon ou du sein qui sont appropriés pour un traitement défini, la méthode comprenant les étapes qui consistent à :
(a) mesurer la quantité de la protéine S100A8/A9 présente dans des échantillons précédemment obtenus de patients atteints d'un cancer du poumon ou du sein ;
(b) évaluer si la quantité mesurée de la protéine S100A8/A9 est inférieure à une valeur standard prédéfinie ou supérieure à ladite valeur standard prédéfinie ; et
(c) sélectionner des patients chez qui la valeur mesurée dans les échantillons est inférieure à la valeur standard prédéfinie pour administration d'un agent de chimiothérapie cytotoxique, et à sélectionner des patients chez qui la valeur mesurée dans les échantillons est supérieure à la valeur standard prédéfinie pour administration d'agents de chimiothérapie qui sont dirigés vers un ou plusieurs composants de l'axe de survie TNF alpha - CXCL1/2 - S100A8/A9 ; dans laquelle lesdits agents de chimiothérapie sont dirigés vers une ou plusieurs cibles sélectionnées parmi le groupe constitué des chimiokines CXCL1/2/3/8 et de leurs récepteurs CXCR1/2, de la cytokine TNF-alpha et de son récepteur TNFR, des facteurs S100A8/A9 et de leurs récepteurs RAGE et TLR4.

2. Méthode selon la revendication 1, dans laquelle les échantillons proviennent de tissus tumoraux.

3. Méthode selon la revendication 1, dans laquelle les échantillons proviennent de sérum.

4. Méthode selon l'une quelconque des revendications 1 à 3, dans laquelle les échantillons ont été obtenus précédemment de patients humains atteints d'un cancer du poumon ou du sein à qui un agent de chimiothérapie cytotoxique a été précédemment administré.

5. Trousse constituée de réactifs pour évaluer la réactivité à des traitements de chimiothérapie d'une patiente humaine atteinte du cancer du sein, ladite trousse comprenant :
(a) des réactifs pour mesurer la quantité de CXCL1/2 et/ou la quantité de TNF-alpha dans un échantillon de la patiente ; et
(b) des réactifs pour mesurer la quantité de S100A/A9 dans un échantillon de la patiente, dans laquelle les réactifs sont des anticorps et/ou des sondes oligonucléotidiques particulières pour CXCL1/2, TNF-alpha et/ou S100A8/A9.

6. Trousse selon la revendication 5, laquelle contient des réactifs pour mesurer la quantité de CXCL1 et de CXCL2 dans l'échantillon.
